# FASCICULE DE BREVET EUROPEEN

(11) **EP 2 507 238 B1**
(45) Date de publication et mention de la délivrance du brevet: **31.07.2013**
(21) Numéro de dépôt: 10801625.4
(22) Date de dépôt: 03.12.2010
(51) Int. Cl.: C07D 471/04, A61K 31/437, A61P 25/28, A61P 25/18, A61P 25/08, A61P 19/10

(54) **DERIVES DE DIPHENYL-PYRAZOLOPYRIDINES, LEUR PREPARATION ET LEUR APPLICATION EN TANT QUE MODULATEURS DU RÉCEPTEUR NUCLÉAIRE NOT**
DIPHENYL-PYRAZOLOPYRIDIN-DERIVATE, HERSTELLUNG DAVON UND VERWENDUNG DAVON ALS NUKLEARER REZEPTOR-NOT-MODULATOREN
DIPHENYL-PYRAZOLOPYRIDINE COMPOUNDS, THEIR PREPARATION AND THEIR APPLICATION AS MODULATORS OF THE NUCLEAR RECEPTOR NOT

(30) Priorité: 04.12.2009 FR 0958651
(43) Date de publication de la demande: 10.10.2012
(73) Titulaire: SANOFI, 75008 Paris (FR)
(72) Inventeur: AUGER, Florian, F-75013 Paris (FR); EVEN, Luc, F-75013 Paris (FR)
(74) Mandataire: Nony
(86) Numéro de dépôt international: PCT/FR2010/052605
(87) Numéro de publication internationale: WO 2011/067544

(56) Documents cités:
- WO-A1-2008/034974
- FR-A1- 2 928 921

## Description

La présente invention se rapporte à des dérivés de diphényl-pyrazolopyridines, à leur préparation et à leur application en thérapeutique dans le traitement ou la prévention de maladies impliquant les récepteurs nucléaires Nurr-1, aussi appelés NR4A2, NOT, TTNUR, RNR-1, et HZF3.

Des composés mis en oeuvre en thérapeutique dans le traitement ou la prévention de telles maladies sont déjà connus dans l'art antérieur, notamment dans W02008/034974 et FR2928921. Toutefois, les composés selon la présente invention se distinguent des composés de ces documents en ce qu'ils consistent en des 2,5-diphényl-pyrazolo[1,5-*a*]pyridines, tandis que les composés de ces documents consistent en des 2,6-diphényl-imidazo[1,2-*a*]pyridines.

La présente invention a pour objet les composés de formule (I) : 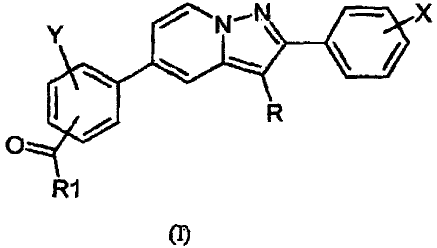 dans laquelle :
R représente un atome d'hydrogène ou d'halogène ou un groupe (C1-C6)alkyle;
X représente un ou plusieurs substituants choisis parmi un atome d'hydrogène ou d'halogène, un groupe (C1-C6)alkyle, halogéno(C1-C6)alkyle, (C1-C6)alcoxy, halogéno (C1 -C6)alcoxy, cyano, hydroxy ou hydroxy(C1-C6)alkyle;
Y représente un atome d'hydrogène, d'halogène ou un groupe (C1-C6)alkyle;
R1 représente un groupe NR2R3 ou OR4;
R2 et R3 représentant indépendamment l'un de l'autre, un atome d'hydrogène, un groupe (C1-C6)alkyle, hydroxy(C1-C6)alkyle ou oxo(C1-C6)alkyle, ou bien R2 et R3 forment avec l'atome d'azote qui les porte un hétérocycle éventuellement substitué par un groupe(C1-C6)alkyle, hydroxy ou oxo,
R4 représente un groupe (C1-C6)alkyle, hydroxy(C1-C6)alkyle ou oxo(C1-C6)alkyle, à l'état de base ou de sol d'addition à un acide.

Les composés de formule (I) peuvent comporter un ou plusieurs atomes de carbone asymétriques. Ils peuvent donc exister sous forme d'énantiomères ou de diastéréoisomères. Ces Enantiomères, diastéréoisoméres, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvates, à savoir sous forme d'associations ou de combinaisons avec une ou plusieurs molécules d'eau ou avec un solvant. De tels hydrates et solvates font également partie de l'invention.

Dans le cadre de la présente invention, on entend par :
- un groupe (Cₓ-Cₜ) : un groupe comprenant entre x et t atomes de carbone ;
- un atome d'halogène : un fluor, un chlore, un brome ou un iode ;
- un groupe alkyle : un groupe aliphatique saturé linéaire, ramifié ou cyclique, éventuellement substitué par un groupe alkyle saturé linéaire, ramifié ou cyclique. A titre d'exemples, on peut citer les groupes méthyle, éthyle, propyle, isopropyle, butyle, isobutyle, tertbutyle, cyclopropyle, cyclobutyl, cyclopentyle, cyclohexyle, méthylcyclopropyle, cyclopropylméthyle etc ;
- un groupe alcoxy : un radical -O-alkyle où le groupe alkyle est tel que précédemment défini ;
- un groupe haloalkyle : un groupe alkyle substitué par un ou plusieurs atomes d'halogène identiques ou différents. A titre d'exemples, on peut citer les groupes CF₃, CH₂CF₃,

CHF₂, CCl₃.
- un groupe hydroxyalkyle : un groupe alkyle substitué par un groupe hydroxyle; à titre d'exemples, on peut citer CH2OH, CH₂CH2OH etc.
- un groupe oxoalkyle : un groupe alkyle substitué par un groupe oxo (C=O); à titre d'exemples, on peut citer CH3CO, CH3COCH2 etc.
- un groupe haloalcoxy : un radical -O-alkyle où le groupe alkyle est tel que précédemment défini et substitué par un ou plusieurs atomes d'halogène identiques ou différents. A titre d'exemples, on peut citer les groupes OCF₃, OCHF₂, OCCl₃
- un groupe aryle : groupe mono ou bicyclique aromatique comportant de 6 à 10 atomes.

A titre d'exemples de groupes aryles, on peut citer phényle et naphthyle.
- un groupe hétérocycle : un groupe cyclique saturé, azoté, éventuellement ponté, comprenant entre 5 et 9 atomes de carbone, au moins un atome d'azote et comprenant éventuellement entre 1 et 3 hétéroatomes additionnels, tels que l'oxygène, l'azote ou le soufre. On peut notamment citer les groupes pipéridinyle, pipérazinyle, pyrrolidinyle, morpholinyle etc.

Parmi les composés de formule (I) objets de l'invention, un premier groupe de composés est constitué des composés pour lesquels :
R représente un atome d'hydrogène ou de chlore,
X représente un ou plusieurs substituants choisis parmi un atome d'halogène, un groupe (C1 - C6)alkyle, halogéno(C1-C6)alkyle, (C1-C6)alcoxy, halogéno(C1-C6)alcoxy, ou cyano,
Y représente un atome d'hydrogène, un atome d'halogène ou un groupe (C1-C6)alkyle;
R1 représente un groupe OR4,
R4 représente un groupe méthyle, à l'état de base ou de sel d'addition à un acide.

Parmi les composés de formule (I) objets de l'invention, un second groupe de composés est constitué des composés pour lesquels :
R représente un atome d'hydrogène ou de chlore,
X représente un ou plusieurs substituants choisis parmi un atome de chlore ou de fluor, un groupe méthyle, trifluorométhyle, méthoxy, trifluorométhoxy ou cyano,
Y représente un atome d'hydrogène, de chlore, de fluor ou un groupe méthyle;
R1 représente un groupe OR4,
R4 représente un groupe méthyle, à l'état de base ou de sel d'addition à un acide.

Parmi les composés de formule (I) objets de l'invention, un troisième groupe de composés est constitué des composés pour lesquels :
R représente un atome d'hydrogène ou de chlore ;
X représente un ou plusieurs substituants choisis parmi un un atome d'halogène, un groupe (C1-C6)alkyle, halogéno(C1-C6)alkyle, (C1-C6)alcoxy, halogéno(C1-C6)alcoxy, ou cyano ;
Y représente un atome d'hydrogène, un atome d'halogène ou un groupe (C1-C6)alkyle;
R1 représente un groupe NR2R3,
R2 et R3 représentent, indépendamment l'un de l'autre, un atome d'hydrogène, un groupe méthyle, éthyle, isopropyle, cyclopropyle, ou bien R2 et R3 forment avec l'atome d'azote qui les porte un groupe morpholinyle ou pyrrolidine éventuellement substitué par un groupe hydroxy, à l'état de base ou de sel d'addition à un acide.

Parmi les composés de formule (I) objets de l'invention, un quatrième groupe de composés est constitué des composés pour lesquels :
R représente un atome d'hydrogène ou de chlore,
X représente un ou plusieurs substituants choisis parmi un atome de chlore ou de fluor, un groupe méthyle, trifluorométhyle, méthoxy, trifluorométhoxy ou cyano,
Y représente un atome d'hydrogène ou de chlore ou un groupe méthyle ;
R1 représente un groupe NR2R3,
R2 et R3 représentent, indépendamment l'un de l'autre, un atome d'hydrogène, un groupe méthyle, éthyle, isopropyle, cyclopropyle, ou bien R2 et R3 forment avec l'atome d'azote qui les porte un groupe morpholinyle ou pyrrolidine éventuellement substitué par un groupe hydroxy, à l'état de base ou de sel d'addition à un acide.

Les combinaisons des groupes un à quatre tels que définis ci dessus font également partie de l'invention.

Parmi les composés de formule (I) objets de l'invention, on peut notamment citer les composés suivants :
- 3-[2-(4-Chlororophényl)pyrazolo[1,5-*a*]pyridin-5-yl]benzoate de méthyle
- 3-[2-(4-Chlorophényl)pyrazolo[1,5-*a*]pyridin-5-yl]benzamide
- 3-[2-(4-Chlorophényl)pyrazolo[1,5-*a*]pyridin-5-yl]-*N*,*N*-diméthylbenzamide
- 3-[2-(4-Chlorophényl)pyrazolo[1,5-*a*]pyridin-5-yl]-*N*-méthylbenzamide
- 3-[2-(4-Fluorophényl)pyrazolo[1,5-*a*]pyridin-5-yl]benzoate de méthyle
- 3-[2-(4-Fluorophényl)pyrazolo[1,5-*a*]pyridin-5-yl]-*N-*méthylbenzamide
- 3-[2-(4-Fluorophényl)pyrazolo[1,5-*a*]pyridin-5-yl]-*N*-isopropylbenzamide
- 3-[2-(4-Fluorophényl)pyrazolo[1,5-*a*]pyridin-5-yl]-*N*,*N*-diméthylbenzamide
- {3-[2-(4-Fluorophényl)pyrazolo[1,5-*a*]pyridin-5-yl]phényl}morpholin-4-yl-méthanone
- 3-[2-(4-Fluorophényl)pyrazolo[1,5-*a*]pyridin-5-yl]-2,*N*-diméthylbenzamide
- 2-Chloro-5-[2-(4-fluorophényl)pyrazolo[1,5-*a*]pyridin-5-yl]-*N-*méthylbenzamide
- 4-Chloro-3-[2-(4-fluorophényl)pyrazolo[1,5-*a*]pyridin-5-yl]-*N*-méthylbenzamide
- 3-[2-(4-Fluorophényl)pyrazolo[1,5-*a*]pyridin-5-yl]-4,*N*-diméthylbenzamide
- 2-Fluoro-4-[2-(4-fluorophényl)pyrazolo[1,5-*a*]pyridin-5-yl]-*N*-méthylbenzamide
- *N*-Cyclopropyl-3-[2-(4-fluorophényl)pyrazolo[1,5-*a*]pyridin-5-yl]benzamide
- {3-[2-(4-Fluorophényl)pyrazolo[1,5-*a*]pyridin-5-yl]phényl}pyrrolidin-1-ylméthanone
- 3-[2-(2,6-Difluorophényl)pyrazolo[1,5-*a*]pyridin-5-yl-méthylbenzamide
- 3-[2-(2-Fluorophényl)pyrazolo[1,5-*a*]pyridin-5-yl]-*N*-méthylbenzamide
- *N*-Méthyl-3-[2-(4-trifluorométhylphényl)pyrazolo[1,5-*a*]pyridin-5-yl]benzamide
- 4-[2-(4-Fluorophényl)pyrazolo[1,5-*a*]pyridin-5-yl]-*N*-méthylbenzamide
- 2-[2-(4-Fluorophényl)pyrazolo[1,5-*a*]pyridin-5-yl]-*N*-méthylbenzamide
- {3-[2-(4-Fluorophényl)pyrazolo[1,5-*a*]pyridin-5-yl]phényl}-(3-hydroxypyrrolidin-1-yl)méthanone
- 3-[2-(2,4-Difluorophényl)pyrazolo[1,5-*a*]pyridin-5-yl]-*N*-méthylbenzamide
- *N*-Méthyl-3-[2-(4-trifluorométhoxyphényl)pyrazolo[1,5-*a*]pyridin-5-yl]benzamide
- 3-[2-(3,4-Difluorophényl)pyrazolo[1,5-*a*]pyridin-5-yl]-*N*-méthylbenzamide
- 3-[2-(3,5-Difluorophényl)pyrazolo[1,5-*a*]pyridin-5-yl]-*N*-méthylbenzamide
- 3-[2-(3-Fluorophényl)pyrazolo[1,5-*a*]pyridin-5-yl]-*N*-méthylbenzamide
- *N*-Méthyl-3-(2-p-tolylpyrazolo[1,5-*a*]pyridin-5-yl)benzamide
- 3-[2-(4-Méthoxyphényl)pyrazolo[1,5-*a*]pyridin-5-yl]-*N*-méthylbenzamide
- 3-[2-(3,4-Diméthylphényl)pyrazolo[1,5-*a*]pyridin-5-yl]-*N*-méthylbenzamide
- 3-[2-(4-Cyanophényl)pyrazolo[1,5-*a*]pyridin-5-yl]-*N*-méthylbenzamide
- 3-[2-(2,3-Difluorophényl)pyrazolo[1,5-*a*]pyridin-5-yl]-*N*-méthylbenzamide
- N-Méthyl-3-(2-o-tolylpyrazolo[1,5-*a*]pyridin-5-yl)benzamide
- 3-[3-Chloro-2-(4-fluorophényl)pyrazolo[1,5-*a*]pyridin-5-yl]-*N*-méthylbenzamide

Conformément à l'invention, on peut préparer les composés de formule générale (I) selon le procédé décrit dans le schéma 1.

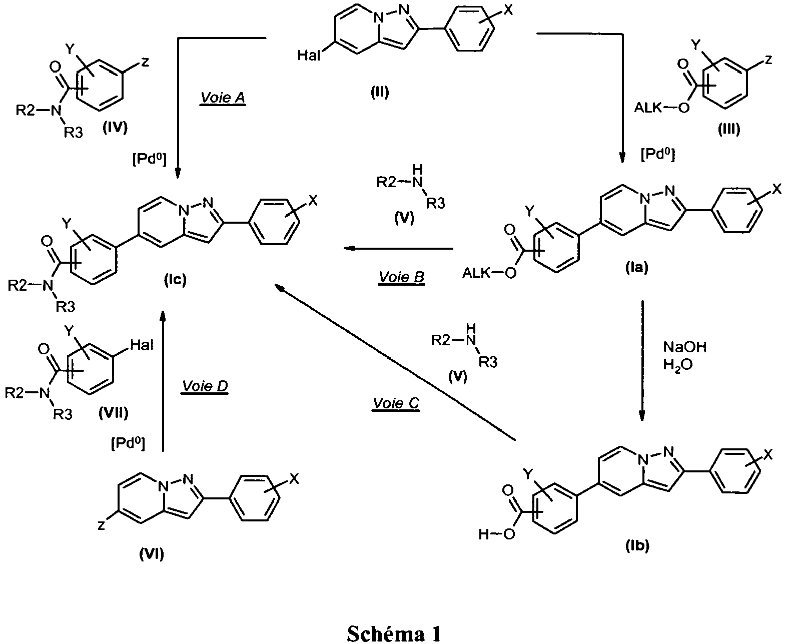

Suivant le schéma 1, on peut préparer les composés de formule générale (Ia), dans laquelle R1 représente OR4, R4 représente un groupe alkyle ALK, R représente un atome d'hydrogène, X et Y sont définis comme précédemment, par une réaction de couplage, catalysée par un métal tel que le palladium, entre un composé de formule générale (II) dans laquelle R représente un atome d'hydrogène, X est défini tel que précédemment et Hal représente un atome d'halogène, et un dérivé de formule générale (III) dans laquelle Y et ALK sont définis tel que précédemment, et Z représente un dérivé de bore.

Suivant le schéma 1, on peut préparer les composés de formule générale (Ib), dans laquelle R1 représente OR4, R représente un atome d'hydrogène, X et Y sont définis comme précédemment et R4 représente un atome d'hydrogène, par une réaction d'hydrolyse des composés de formule générale (Ia) par une base telle que la soude dans un milieu hydro-alcoolique.

Suivant le schéma 1 *voie A*, on peut préparer les composés de formule générale (Ic) dans laquelle R1 représente NR2R3, R représente un atome d'hydrogène et X, Y, R2 et R3 sont définis comme précédemment, par une réaction de couplage, catalysée par un métal tel que le palladium, entre un composé de formule générale (II) dans laquelle R représente un atome d'hydrogène, X est défini tel que précédemment et Hal représente un atome d'halogène et un dérivé de formule générale (IV) dans laquelle Y, R2 et R3 sont définis tel que précédemment et Z représente un dérivé de bore.

Suivant le schéma 1 *voie B*, on peut préparer les composés de formule générale (Ic) dans laquelle R1 représente NR2R3, R représente un atome d'hydrogène et X, Y, R2 et R3 sont définis comme précédemment, par une réaction entre un composé de formule générale (Ia), dans laquelle R1 représente OR4, R4 représente un groupe alkyle ALK, R représente un atome d'hydrogène, X, Y sont définis comme précédemment, et une amine de formule générale (V) dans laquelle R2 et R3 sont définis comme précédemment, en présence de triméthylaluminium en solution ou bien complexé avec une amine tertiaire telle que le DABCO selon la méthode décrite par D. Glynn, D. Bernier, S. Woodward dans Tetrahedron Letters, 2008, 49, 5687-5688.

Suivant le schéma 1 *voie C,* on peut préparer les composés de formule générale (Ic) dans laquelle R1 représente NR2R3, R représente un atome d'hydrogène et X, Y, R2 et R3 sont définis comme précédemment par une réaction entre un composé de formule générale (Ib), dans laquelle R1 représente OR4, R représente un atome d'hydrogène, X et Y sont définis comme précédemment et R4 représente un atome d'hydrogène, et une amine de formule générale (V) dans laquelle R2 et R3 sont définis comme précédemment, en présence d'un activateur d'acide tel que le chloroformate d'isobutyle.

Suivant le schéma 1 *voie D*, on peut préparer les composés de formule générale (le) dans laquelle R1 représente NR2R3, R représente un atome d'hydrogène et X, Y, R2 et R3 sont définis comme précédemment par une réaction de couplage, catalysée par un métal tel que le palladium, entre un composé de formule générale (VI) dans laquelle R représente un atome d'hydrogène, X est défini tel que précédemment et Z représente un dérivé de bore et un dérivé de formule générale (VII) dans laquelle Y, R2 et R3 sont définis tel que précédemment et Hal. représente un atome d'halogène.

On peut également préparer les composés de formule générale (Ic) dans laquelle R2 et R3 représentent chacun un atome d'hydrogène selon le procédé décrit dans le schéma 2.

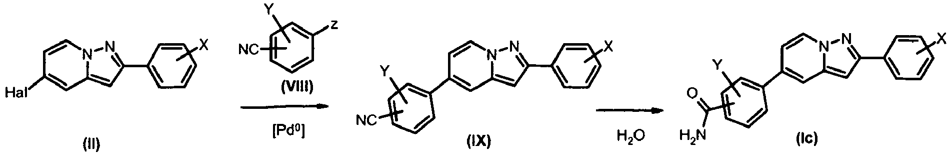

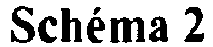

Dans le schéma 2, les composés de formule générale (Ic) dans laquelle R1 représente NH2, R représente un atome d'hydrogène et X et Y sont définis comme précédemment, peuvent être obtenus par hydrolyse des nitriles de formule générale (IX), par exemple au moyen d'eau oxygénée en présence de base. Les composés de formule générale (IX) peuvent être obtenus par une réaction de couplage, catalysée par un métal tel que le palladium, entre un composé de formule générale (II) dans laquelle R représente un atome d'hydrogène, X est défini tel que précédemment et Hal représente un atome d'halogène et un dérivé de formule générale (VIII) dans laquelle Y est défini tel que précédemment, CN représente un groupe cyano et Z représente un dérivé de bore.

Conformément à l'invention, on peut préparer les composés de formule générale (I) selon le procédé décrit dans le schéma 3.

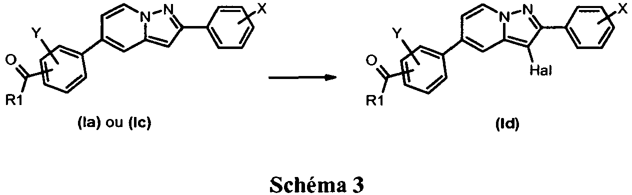

Suivant le schéma 3, on peut préparer les composés de formule générale (Id) dans laquelle X, Y et R1 sont définis comme précédemment et R représente un atome d'halogène Hal par halogénation électrophile des composés (Ia) ou (Ic), par exemple par chloration, au moyen d'un agent tel que la *N*-chlorosuccinimide.

Conformément à l'invention, on peut préparer les composés de formule générale (II) et (VI) selon le procédé décrit dans le schéma 4.

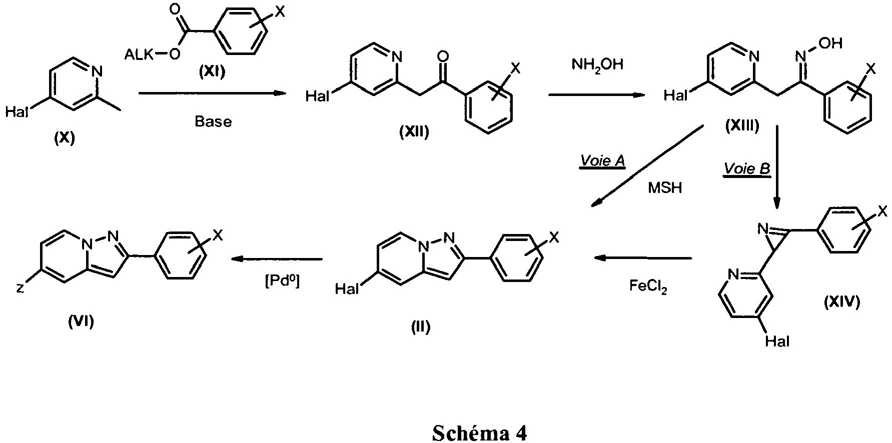

Dans le schéma 4 *voie* A, les composés de formule générale (II) dans laquelle X est défini comme précédemment, R représente un atome d'hydrogène et Hal représente un atome d'halogène peuvent être préparés par action du O-(mésitylènesulfonyl)hydroxylamine (MSH) sur un composé de formule générale (XIII) dans laquelle X et Hal sont définis comme précédemment, par exemple selon la méthode décrite par Y. Tamura, J.-H. Kim, Y. Miki, H. Hayashi, M. Ikeda, dans J.Het. Chem., 1975, 12, 481.

Dans le schéma 4 *voie B*, on peut également préparer les composés de formule générale (II) dans laquelle X est défini comme précédemment, R représente un atome d'hydrogène et Hal représente un atome d'halogène par transformation des composés de formule générale (XIII) en composés de formule générale (XIV) dans laquelle X et Hal sont définis comme précédemment, par action d'un anhydride d'acide tel que l'anhydride trifluoracétique en présence d'une base telle que la triéthylamine, puis cyclisation en composés de formule générale (II) en présence d'un catalyseur comme le chlorure ferreux, par exemple selon la méthode décrite par K.S. Gudmundsson dans Bioorg. Med. Chem., 2005, 13, 5346.

Les composés (XIII) peuvent être obtenus à partir des composés (XII) par action de l'hydroxylamine. Les composés (XII) peuvent être obtenus à partir des picolines de formule générale (X) et des esters de formule générale (XI) dans laquelle X est défini comme précédemment et ALK représente un groupement alkyle, en présence d'une base forte, par exemple selon la méthode décrite par K.S. Gudmundsson dans Bioorg. Med Chem., 2005, 13, 5346.

Enfin, on peut préparer les composés (VI) dans lesquels Z représente un dérivé de bore suivant le schéma 3 par une réaction de couplage, par exemple du bis(pinacolato)dibore, sur les composés (II), catalysée par un métal tel que le palladium selon la méthode décrite par E.F. DiMauro, R.Vitullo, J.Org.Chem., 2006, 71(10), 3959.

Dans les schémas 1, 2, 3 et 4, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

L'invention, selon un autre de ses aspects, a également pour objet le composé de formule (VI-1). Ce composé est utile comme intermédiaire de synthèse des composés de formule (I).

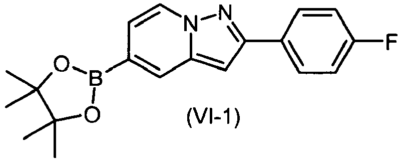

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

### Exemple 1 : 3-[2-(4-Chlororophényl)pyrazolo[1,5-a]pyridin-5-yl]benzoate de méthyle (composé 1 du tableau)

### 1.1 2-(4-Bromopyridin-2-yl)-1-(4-chlorophényl)éthanone

Sous courant d'azote, 5 g (29,07 mmol) de 4-bromo-2-méthylpyridine et 11,27 g (61,04 mmol) de 4-chlorobenzoate d'éthyle sont placés dans un ballon et dissous dans 50 ml de tétrahydrofurane anhydre. On refroidit à 5°C et on ajoute goutte à goutte 70 mL (70 mmol) d'une solution d'hexaméthyldisilazane de lithium (1M dans le tétrahydrofurane). Après addition, le mélange est agité à température ambiante pendant 2h, refroidi à 5°C, puis 100 mL d'eau sont additionnés progressivement. Le milieu est ensuite dilué avec 250 mL d'acétate d'éthyle et 100 mL d'eau. La phase organique est séparée, la phase aqueuse est extraite deux fois avec 100 ml d'acétate d'éthyle. Les phases organiques sont ensuite réunies, séchées sur sulfate de sodium et filtrées. On ajoute ensuite au filtrat 15 g de silice, concentre sous pression réduite. La poudre obtenue est utilisée comme dépôt solide pour une chromatographie sur gel de silice avec pour éluant un mélange de cyclohexane et d'acétate d'éthyle (9/1). 8,4 g (93%) de composé sont obtenus sous forme d'une poudre jaune.
LC-MS: M+H = 310
RMN-¹H (DMSO) δ (ppm) : 4,6 (s, 2H) ; 6,4 (s, 1H) ; 7,4 (s, 1H) ; de 7,5 à 7,6 (m, 6H) ; 7,7 (s, 1H) ; 7,9 (d, 2H) ; 8,1 (d,2H) ; 8,3 (d, 1H) ; 8,4 (d, 1H) ; 15,0 (s, 1H) (mélange *cétone* / *énol:* 40 / 60).

### 1.2 2-(4-Bromopyridin-2-yl)-1-(4-chlorophényl)éthanone oxime

On place dans un ballon 8,4 g (27,05 mmol) de 2-(4-bromopyridin-2-yl)-1-(4-chlorophényl)éthanone dans 150 mL d'éthanol. On ajoute 22 mL (272,56 mmol) de pyridine et 7,5 g (107,93 mmol) d'hydroxylamine monochlorhydrate Le mélange est ensuite agité pendant 5 heures à température ambiante puis le milieu réactionnel est concentré sous pression réduite jusqu'à obtention d'un solide pâteux jaune que l'on reprend avec 400mL d'acétate d'éthyle et 400 mL d'eau. La phase organique est séparée, la phase aqueuse est extraite trois fois avec 200 mL d'acétate d'éthyle. Les phases organiques sont ensuite réunies, séchées sur sulfate de sodium et filtrées. Le filtrat est concentré sous pression réduite : on obtient 8,1 g (91,9%) de composé sous forme d'une poudre bleue.
LC-MS : M+H = 325
RMN-¹H (DMSO) δ (ppm) : 4,3 (s, 2H) ; 7,45 (m, 2H) ; 7,50 (d, 1H) ; 7,55 (s, 1H) ; 7,75 (m, 2H) ; 8,35 (d, 1H) ; 11,65 (s, 1 H).

### 1.3. 5-Bromo-2-(4-chlorophényl)pyrazolo[1,5-a]pyridine

On place dans un ballon 12,9 g (45,21 mmol) de *O*-(2-mésitylènesulfonyl)acétohydroxamate d'éthyle dans 30 mL de 1,4-dioxane. On refroidit à 0°C et on ajoute 13,5 mL (156,60 mmol) d'acide perchlorique (70% dans l'eau). On ajoute ensuite 10 mL de 1,4-dioxane puis le milieu est agité vigoureusement pendant 2h30 minutes à 0°C. Le milieu est ensuite versé dans 350mL d'eau glacée. On laisse le milieu vers 0°C pendant 10 minutes puis on récupère par filtration sur verre fritté le solide blanc formé (ne pas sécher totalement, le produit est potentiellement explosif à l'état sec). Le solide pâteux blanc obtenu est lavé avec 350 mL d'eau glacée puis est repris avec 250mL de 1,2-dichloroéthane et 150mL de saumure refroidie vers 5°C. On récupère la phase organique que l'on filtre sur cartouche hydrophobe. On récupère le filtrat que l'on ajoute goutte à goutte sur une solution refroidie vers 0°C de 8,1g (24,88 mmol) de 2-(4-bromopyridin-2-yl)-1-(4-chlorophényl)éthanone oxime (composé obtenu dans l'étape 1.2) dans 150mL de 1,2-dichloroéthane.

Après l'ajout, on laisse revenir et on agite à température ambiante pendant 3 heures. On ajoute ensuite successivement au milieu 250mL de dichlorométhane, 200mL d'eau et 100 mL d'une solution aqueuse NaOH (1N). On laisse agiter puis on décante. La phase organique est séparée et la phase aqueuse est extraite avec 2 fois 200 mL de dichlorométhane. Les phases organiques sont ensuite réunies, filtrées sur cartouche hydrophobe (Colonne d'extraction liquide/liquide 70 mL, Radleys®) puis mélangées avec 15 g de silice. Le filtrat est ensuite concentré sous pression réduite : on obtient une poudre marron que l'on utilise comme dépôt solide pour une chromatographie sur gel de silice en éluant avec un mélange de cyclohexane et de dichlorométhane (1/1). On obtient 5,8 g (75%) de composé sous forme d'un solide cotonneux légèrement jaune.
LC-MS : M+H = 307.
RMN-¹H (DMSO) δ (ppm) : 7,0 (d, 1H) ; 7,1 (s, 1H) ; 7,6 (d, 2H) ; 8,0 (s, 1H) ; 8,1 (d, 2H) ; 8,7 (d, 1H).

### 1.4 3-[2-(4-Chlororophényl)pyrazolo[1,5-a]pyridin-5-yl]benzoate de méthyle

On place dans un ballon 0,235 g (0,76 mmol) de 5-bromo-2-(4-chlorophényl)pyrazolo[1,5-*a*]pyridine obtenu dans l'étape 1.3, 0,165 g (0,92 mmol) d'acide 3-méthoxycarbonylphénylboronique , 0,750 g (2,30 mmol) de carbonate de césium et 0,065 g (0,08 mmol) de [1,1'-bis(diphenylphosphino)ferrocène]dichloropalladium (II) en présence de 5 mL d'un mélange THF-eau (9/1). Le milieu est ensuite porté à 70°C pendant 1 h30 puis le milieu est remis à température ambiante et dilué avec 30 mL de dichlorométhane et 30 mL d'eau. Le milieu biphasique est filtré sur cartouche hydrophobe (Colonne d'extraction liquide/liquide 70 mL, Radleys®) puis le filtrat concentré sous pression réduite : le résidu obtenu est chromatographié sur gel de silice en éluant avec un mélange de cyclohexane et d'acétate d'éthyle (8/2). On obtient 0,200 g (72%) de composé attendu sous forme d'une poudre beige.
Point de fusion (°C) : 180-182
LC-MS : M+H = 363
RMN-¹H (DMSO) δ (ppm) : 3,95 (s, 3H) ; 7,20 (s, 1H) ; 7,35 (d, 1H) ; 7,60 (d, 2H) ; 7,70 (t, 1H) ; de 8,00 à 8,20 (m, 5H) ; 8,35 (s, 1H) ; 8,85 (d, 1H).

### Exemple 2 : 3-[2-(4-Chlorophényl)pyrazolo[1,5-a]pyridin-5-yl]benzamide (composé 2 du tableau)

### 2.1 3-[2-(4-Chlorophényl)pyrazolo[1,5-a]pyridin-5-yl]benzonitrile

0,850 g (2,76 mmol) de 5-bromo-2-(4-chlorophényl)pyrazolo[1,5-a]pyridine obtenu selon le protocole de l'étape 1.3 sont placés dans un ballon avec 0,490 g (3,33 mmol) d'acide 3-cyanophénylboronique, 2,70 g (8,29 mmol) de carbonate de césium et 0,225 g (0,26 mmol) de [1,1'-bis(diphenylphosphino)ferrocène]dichloropalladium (II) en présence de 20 mL d'un mélange THF-eau (9/1). Le milieu est ensuite porté à 75°C pendant 3 h avant de rajouter 0,245 g (1,66 mmol) d'acide 3-cyanophénylboronique, 1,35 g (4,14 mmol) de carbonate de césium et 0,115 g (0,14 mmol) de [1,1'-bis(diphenylphosphino)ferrocène]dichloropalladium (II) et d'agiter le milieu à 75°C pendant 1h30. Le milieu est ensuite dilué avec 100 mL d'acétate d'éthyle et 100 mL d'eau. La phase organique est ensuite récupérée et la phase aqueuse est extraite deux fois avec 100 mL d'acétate d'éthyle. Les phases organiques sont ensuite réunies avant d'être séchées sur sulfate de sodium et filtrées. Le filtrat est alors concentré sous pression réduite puis le résidu obtenu est dissout dans du tétrahydrofuranne et concentré sous pression réduite après ajout de 10g de silice. Le résidu est chromatographié sur gel de silice en éluant avec un mélange de cyclohexane et d'acétate d'éthyle (8/2). On obtient 0,185 g (20,2%) de composé attendu sous forme d'une poudre blanche.
LC-MS : M+H = 330
RMN-¹H (DMSO) δ (ppm) : 7,19 (s, 1H) ; 7,37 (dd, 1H) ; 7,56 (m, 2H) ; 7,74 (t, 1H) ; 7,90 (m, 1H) ; 8,06 (m, 2H) ; de 8,15 à 8,24 (m, 2H) ; 8,35 (m, 1H) ; 8,82 (d, 1H).

### 2.2 3-[2-(4-Chlorophényl)pyrazolo[1,5-a]pyridin-5-yl]benzamide

0,150 g (0,45 mmol) de-[2-(4-chlorophényl)pyrazolo[1,5-*a*]pyridin-5-yl]benzonitrile obtenu dans l'étape 2.1 sont placés dans un ballon avec 5 mL de diméthylsulfoxide anhydre. Le milieu est alors refroidi vers 10°C et 0,100 mL (1,17 mmol) d'une solution d'eau oxygénée (35% dans l'eau) et 0,035 g (0,25 mmol) de carbonate de potassium sont ajoutés. Le milieu est remis progressivement à température ambiante et agité pendant 1 heure. On refroidit ensuite le milieu vers 5°C pour rajouter 0,500 mL (5,85 mmol) d'eau oxygénée et 0,250 g (1,78 mmol) de carbonate de potassium. Le milieu est ensuite agité pendant 1h30 à température ambiante avant d'être dilué dans 50 mL d'eau. On filtre le milieu sur verre fritté et on récupère une poudre que l'on chromatographie (par dépôt solide) sur gel de silice en éluant avec un mélange de dichlorométhane et de méthanol (9/1). On obtient 0,090 g (56,8%) de composé attendu sous forme d'une poudre blanche.
Point de fusion (°C) : 283-285
LC-MS : M+H = 348
RMN-¹H (DMSO) δ (ppm) : 7,17 (s, 1H) ; 7,36 (dd, 1H) ; 7,47 (s, 1H) ; 7,56 (m, 2H) ; 7,61 (t, 1H) ; 7,94 (m, 1H) ; 8,00 (m, 1H) ; 8,07 (m, 2H) ; 8,11 (m, 1H) ; 8,15 (s, 1H) ; 8,32 (m, 1H) ; 8,82 (d, 1H).

### Exemple 3: 3-[2-(4-Chlorophényl)pyrazolo[1,5-a]pyridin-5-yl]-N,N-diméthylbenzamide (composé 3 du tableau)

Sous courant d'azote, 0,900mL (1,80 mmol) d'une solution de diméthylamine (2M dans tetrahydrofuranne) et 8 mL de toluène sous placés dans un ballon. Le milieu est ensuite refroidi vers 0°C puis 0,900 mL (1,80 mmol) d'une solution de triméthylaluminium (2M dans toluène) sont ajoutés goutte à goutte. Après l'ajout, le milieu est agité vers 0°C pendant 25 minutes avant d'ajouter 0,200 g (0,55 mmol) de 3-[2-(4-chlororophényl)pyrazolo[1,5-a]pyridin-5-yl]benzoate de méthyle obtenu à l'étape 2.1. Le milieu est ensuite porté à 90°C pendant 3 heures avant d'être refroidi vers 0°C. Le milieu est alors hydrolyse par ajout goutte à goutte de 10 mL d'une solution d'acide chlorhydrique (1N). Après l'ajout, le milieu est remis à température ambiante puis dilué avec 60 mL de dichlorométhane et 60 mL d'eau. Le pH de la phase aqueuse est amené vers 11 à l'aide d'une solution de soude (1N) puis on filtre le milieu biphasique obtenu sur verre fritté garni de Célite. On récupère le filtrat que l'on passe sur cartouche hydrophobe (Colonne d'extraction liquide/liquide 70 mL, Radleys®). Le filtrat est récupéré et concentré sous pression réduite après ajout de 1,2 g de silice. Le résidu obtenu est chromatographié sur gel de silice en éluant avec un mélange de cyclohexane et d'acétate d'éthyle (3/7).

On obtient 0,121 g (58,4%) de composé attendu sous forme d'une poudre blanche.
Point de fusion (°C) : 175-177
LC-MS : M+H = 376
RMN-¹H (DMSO) δ (ppm) : 3,02 (d, 6H) ; 7,15 (s, 1H) ; 7,35 (dd, 1H) ; 7,46 (m, 1H) ; de 7,50 à 7,67 (m, 3H) ; 7,85 (m, 1H) ; 7,91 (m, 1H) ; de 8,00 à 8,15 (m, 3H) ; 8,80 (d, 1H).

### Exemple 4: 3-[2-(4-Chlorophényl)pyrazolo[1,5-a]pyridin-5-yl]-N-méthylbenzamide (composé 4 du tableau)

On procède suivant le mode opératoire décrit dans l'exemple 3. à partir de 0,200 g (0,55 mmol) de 3-[2-(4-chlororophényl)pyrazolo[1,5-*a*]pyridin-5-yl]benzoate de méthyle obtenu à l'étape 3.1, 0,900 mL (1,80 mmol) d'une solution de méthylamine (2M dans tetrahydrofuranne) et 0,900 mL (1,80 mmol) d'une solution de triméthylaluminium (2M dans toluène) dans 8 mL de toluène. Après chromatographie sur gel de silice en éluant avec un mélange de cyclohexane et d'acétate d'éthyle (1/1), on récupère 0,151 g (75,6%) de composé attendu sous forme d'une poudre blanche.
Point de fusion (°C) : 234-236
LC-MS : M+H = 362
RMN-¹H (DMSO) δ (ppm) : 2,85 (d, 3H) ; 7,18 (s, 1H) ; 7,35 (m, 1H) ; de 7,51 à 7,68 (m, 3H) ; 7,90 (m, 1H) ; 8,00 (m, 1H) ; de 8,02 à 8,12 (m, 3H) ; 8,28 (m, 1H) ; 8,62 (m, 1H) ; 8,82 (d, 1H).

### Exemple 5: 3-[2-(4-fluorophényl)pyrazolo[1,5-a]pyridin-5-yl]benzoate de méthyle (composé 5 du tableau)

### 5.1 2-(4-bromopyridin-2-yl)-1-(4-fluorophényl)éthanone

Sous courant d'azote, 5,0 g (29,07 mmol) de 4-bromo-2-picoline et 10,2 g (60,95 mmol) de 4-fluorobenzoate d'éthyle sont placés dans un ballon et dissous dans 50 mL de tétrahydrofurane anhydre. On refroidit à 0°C et on ajoute goutte à goutte 70 mL (70 mmol) d'une solution d'hexaméthyldisilazane de lithium (1M dans le tétrahydrofurane). Après addition, le mélange est agité à température ambiante pendant 2h, refroidi à 5°C, puis 100 mL d'eau sont additionnés progressivement. Le milieu est ensuite dilué avec 250 mL d'acétate d'éthyle et 100 mL d'eau. La phase organique est séparée, la phase aqueuse est extraite deux fois avec 100 mL d'acétate d'éthyle. Les phases organiques sont ensuite réunies, séchées sur sulfate de sodium et filtrées. On ajoute ensuite au filtrat 15 g de silice, agite puis concentre sous pression réduite. La poudre obtenue est utilisée comme dépôt solide pour une chromatographie sur gel de silice avec pour éluant un mélange de cyclohexane et d'acétate d'éthyle (9/1). 7,5 g (88%) de composé sont obtenus sous forme d'une poudre jaune.
LC-MS : M+H = 294 (ratio *cétone* / *énol*: 43 / 57)
RMN-¹H (DMSO) δ (ppm) : 4,56 (s, 2H) ; 6,34 (s, 1H) ; de 7,23 à 7,40 (m, 5H) ; 7,53 (d, 1H) ; 7,56 (m, 1H) ; 7,70 (d, 1H) ; de 7,81 à 7,92 (m, 2H) ; de 8,04 à 8,16 (m,2H) ; 8,29 (d, 1H) ; 8,37 (d, 1H) ; 15,0 (s, 1H).

### 5.2 2-(4-bromopyridin-2-yl)-1-(4-fluorophényl)éthanone oxime

7,5 g (24,26 mmol) de 2-(4-bromopyridin-2-yl)-1-(4-fluorophényl)éthanone sont placés dans un ballon contenant 100 mL d'éthanol absolu. 20 mL (247,78 mmol) de pyridine et 7,08 g (101,88 mmol) d'hydroxylamine monochlorhydrate sont ajoutés avant de laisser agiter le milieu pendant 3h à température ambiante. L'éthanol est ensuite évaporé sous vide et le résidu obtenu est repris avec 250 mL d'eau et 250 mL d'acétate d'éthyle. La phase organique est séparée, puis on extrait la phase aqueuse 5 fois avec 150 mL d'acétate d'éthyle. Les phases organiques sont ensuite réunies, séchées sur sulfate de sodium et concentrée sous vide. 7,82 g de composé sont obtenus.
LC-MS : M+H = 309
RMN 1H (DMSO-d6, δ en ppm): 4,26 (s, 2H) ; 7,19 (t, 2H) ; 7,50 (m, 2H) ; 7,75 (m, 2H) ; 8,33 (d, 1H) ; 11,50 (s, 1H). (obtention de l'oxime (E)).

### 5.3 5-bromo-2-(4-fluororophényl)pyrazolo[1,5-a]pyridine

7,82 g (25,50 mmol) de 2-(4-bromopyridin-2-yl)-1-(4-fluorophényl)éthanone oxime sont placés dans un ballon et dissous dans 400 mL de 1,2-dichloroéthane. Une solution de *O-*(mésitylènesulfonyl)hydroxylamine (0,27 M dans le 1,2-dichloroéthane - composé obtenu selon le protocole décrit en 1.3) est ajoutée goutte à goutte au milieu refroidi vers 0°C. Après l'ajout, le milieu est agité à température ambiante pendant 1h30. Le milieu est ensuite dilué avec 200mL d'eau et 200mL d'une solution de soude (1N). Le milieu biphasique est agité puis décanté. La phase organique est séparée, puis la phase aqueuse est extraite 4 fois avec 200 mL de dichlorométhane. Les phases organiques sont ensuite réunies, séchées sur sulfate de sodium et filtrées. On ajoute ensuite au filtrat 15 g de silice puis concentre sous pression réduite. La poudre obtenue est utilisée comme dépôt solide pour une chromatographie sur gel de silice avec pour éluant un mélange de cyclohexane et de dichlorométhane (1/1). 5,06 g (68%) de composé sont obtenus sous forme d'une poudre cotonneuse blanche.
LC-MS : M+H = 291
RMN 1H (DMSO-d6, δ en ppm): de 7,00 à 7,10 (m, 2H) ; 7,45 (m, 2H) ; 8,05 (m, 3H) ; 8,70 (d, 1H).

### 5.4 3-[2-(4-fluorophényl)pyrazolo[1,5-a]pyridin-5-yl]benzoate de méthyle

Sous courant d'azote sont introduits 0,400 g (1,37 mmol) de 5-bromo-2-(4-fluorophényl)pyrazolo[1,5-*a*]pyridine obtenu à l'étape 5.3., 0,300 g (1,67 mmol) d'acide 3-méthoxycarbonylphénylboronique, 1,330 g (4,08 mmol) de carbonate de césium dans 5 mL d'un mélange 9/1 de tétrahydrofurane et d'eau. 0,11 g (0,13 mmol) de [1,1'-bis(diphenylphosphino)ferrocène]dichloropalladium (II) sont ajoutés et le milieu est chauffé à 70°C pendant 4 heures. Le milieu est ensuite remis à température ambiante puis dilué avec 40 mL de dichlorométhane et 40 mL d'eau. Le milieu est ensuite filtré sur cartouche hydrophobe (Colonne d'extraction liquide/liquide 70 mL, Radleys®), la phase organique est récupérée et concentrée sous pression réduite après avoir ajouté 2 g de silice. Le résidu par chromatographie sur gel de silice en éluant avec un mélange de cyclohexane et d'acétate d'éthyle (9/1). On obtient 0,340 g (71%) de produit attendu sous la forme d'une poudre blanche.
Point de fusion (°C) : 162-164
LC-MS : M+H = 347
RMN-¹H (DMSO) δ (ppm) : 3,95 (s, 3H) ; 7,15 (s, 1H) ; de 7,30 à 7,38 (m, 3H) ; 7,70 (t, 1H) ; de 8,00 à 8,15 (m, 5H) ; 8,35 (m, 1H) ; 8,80 (d, 1H).

### Exemple 6 3-[2-(4-Fluorophényl)pyrazolo[1,5-a]pyridin-5-yl]-N-méthylbenzamide (composé 6 du tableau)

On procède suivant le mode opératoire décrit dans l'exemple3. à partir de 0,200 g (0,58 mmol) de 3-[2-(4-fluorophényl)pyrazolo[1,5-a]pyridin-5-yl]benzoate de méthyle obtenu à l'étape 7.4, 1,00 mL (2,00 mmol) d'une solution de méthylamine (2M dans tetrahydrofuranne) et 1,00 mL (2,00 mmol) d'une solution de triméthylaluminium (2M dans toluène) dans 8 mL de toluène. Après chromatographie sur gel de silice en éluant avec un mélange de cyclohexane et d'acétate d'éthyle (1/1), on récupère 0,235 g (67,7%) de composé attendu sous forme d'une poudre blanche.
Point de fusion (°C) : 214-216
LC-MS : M+H = 346
RMN-¹H (DMSO) δ (ppm) : 2,85 (d, 3H) ; 7,15 (s, 1H) ; de 7,26 à 7,46 (m, 3H) ; 7,62 (m, 1H) ; 7,90 (m, 1H) ; 8,00 (m, 1H) ; de 8,05 à 8,21 (m, 3H) ; 8,29 (m, 1H) ; 8,60 (m, 1H) ; 8,82 (d, 1H).

### Exemple 7: 3-[2-(4-Fluorophényl)pyrazolo[1,5-a]pyridin-5-yl]-2,N-diméthyl benzamide (composé 10 du tableau)

### 7.1 2-(4-Fluorophényl)-5-(4,4,5,5-tétraméthyl-[1,3,2]dioxaborolan-2-yl)pyrazolo[1,5-a]pyridine (Composé VI-1)

1,00 g (3,43 mmol) de 5-bromo-2-(4-fluorophényl)pyrazolo[1,5-a]pyridine obtenus tel qu'en 7.3 sont placés en présence de 1,05 g (4,13 mmol) de bis(pinacolato)dibore, de 1,00 g (10,19 mmol) d'acétate de potassium et 0,280 g (0,34 mmol) de [1,1'-bis(diphénylphosphino)ferrocène]dichloropalladium (II) dans 14 mL de dioxanne. Le milieu obtenu est irradié par micro-onde à 140°C pendant 20 minutes avant d'être dilué avec 100 mL de dichlorométhane et 100 mL d'eau. On filtre ensuite le milieu biphasique sur cartouche hydrophobe (Colonne d'extraction liquide/liquide 70 mL, Radleys®). La phase organique est récupérée et concentrée sous pression réduite après avoir ajouté 4 g de silice. Le résidu obtenu est chromatographié sur gel de silice en éluant avec un mélange de cyclohexane et d'acétate d'éthyle (9/1).

On obtient 0,992 g (85,4%) de composé attendu sous forme d'une poudre rosée.
LC-MS : M+H = 338 (dégradation sur colonne en acide boronique M+H= 257)
RMN-¹H (DMSO) δ (ppm) : 1,35 (s, 12H) ; 7,00 (m, 1H) ; 7,19 (s, 1H) ; 7,34 (t, 2H) ; 8,05 (m, 3H) ; 8,69 (d, 1 H).

### 7.2 3-Bromo-2,N-diméthylbenzamide

0,500 g (2,33 mmol) d'acide 3-bromo-2-méthylbenzoïque sont placés dans un ballon en présence de 1,51 mL (10,83 mmol) de triéthylamine, 0,408 g (3,02 mmol) de N-hydroxybenzotriazole monohydrate, 0,579 g (3,02 mmol) de 1-(3-diméthylaminopropyl)-3-éthylcarbodiimide monochlorhydrate et 5 mL de dichlorométhane. Le milieu est agité à température ambiante pendant 1 heure puis on ajoute 1,51 mL (3,02 mmol) d'une solution de méthylamine (2M dans tétrahydrofuranne). Le milieu est agité toute une nuit à température ambiante puis on rajoute 0,5 mL (1 mmol) de solution de méthylamine et on laisse agiter pendant toute une nuit. Le milieu est ensuite concentré sous pression réduite puis on ajoute au milieu 5 mL de dichlorométhane et 0,390 mL (2,99 mmol) de chloroformate d'isobutyle. Le milieu est à nouveau agité toute une nuit avant d'être dilué avec 7 mL de dichlorométhane et 7 mL d'eau. Le milieu est alors filtré sur cartouche hydrophobe (Colonne d'extraction liquide/liquide 70 mL, Radleys®). La phase organique est récupérée et concentrée sous pression réduite.

On obtient 0,298 g (56,2%) de composé attendu sous forme d'une poudre blanche.
LC-MS : M+H = 228
RMN ¹H (DMSO) δ (ppm): 2,31 (s, 3H) ; 2,80 (d, 3H) ; de 7,05 à 7,35 (m, 2H) ; 7,68 (m, 1 H) ; 8,32 (s él., 1 H).

### 7.3 3-[2-(4-Fluorophényl)pyrazolo[1,5-a]pyridin-5-yl]-4,N-diméthylbenzamide

On place 0,150g (0,44 mmol) de 2-(4-fluorophényl)-5-(4,4,5,5-tétraméthyl-[1,3,2]dioxaborolan-2-yl)pyrazolo[1,5-a]pyridine obtenu dans l'étape 7.1, 0,144 g (0,63 mmol) de 3-bromo-2,*N*-diméthylbenzamide obtenu dans l'étape 10.2 avec 0,434 g (1,33 mmol) de carbonate de césium et 0,036 g (0,044 mmol) de [1,1'-bis(diphénylphosphino)ferrocène]dichloropalladium (II) dans 5 mL d'un mélange 9/1 de tétrahydrofurane et d'eau. Le milieu est agité à 60°C pendant toute une nuit. Le milieu est ensuite dilué avec 50 mL de dichlorométhane et 50 mL d'eau. Le milieu biphasique est alors filtré sur cartouche hydrophobe (Colonne d'extraction liquide/liquide 70 mL, Radleys®). La phase organique est récupérée et concentrée sous pression réduite après avoir ajouté 1,5 g de silice. Le résidu obtenu est chromatographié sur gel de silice en éluant avec un mélange de cyclohexane et d'acétate d'éthyle (1/1).

On obtient 0,103 g (65%) de composé attendu sous forme d'une poudre blanche.
Point de fusion (°C) : 240-242
LC-MS : M+H = 360
RMN ¹H (DMSO) δ (ppm): 2,37 (s, 3H) ; 2,80 (d, 3H) ; 6,86 (d, 1H) ; 7,10 (s, 1H) ; de 7,33 à 7,44 (m, 5H) ; 7,63 (s, 1H) ; 8,10 (m, 2H) ; 8,28 (s, 1H) ; 8,76 (d, 1H).

### Exemple 8: N-Cyclopropyl-3-[2-(4-fluorophényl)pyrazolo[1,5-a]pyridin-5-yl]benzamide (composé 15 du tableau)

0,0382 g (0,67 mmol) de cyclopropylamine sont dilués avec 10 mL de tétrahydrofuranne anhydre. On ajoute ensuite progressivement 0,0859 g (0,33 mmol) de DABAL (adduit double de triméthylaluminium avec du 1,4-diazabicyclo[2.2.2]octane) avant de laisser agiter le milieu pendant 1 heure. 0,145 g (0,42 mmol) de 3-[2-(4-fluorophényl)pyrazolo[1,5-a]pyridin-5-yl]benzoate de méthyle obtenu selon le protocole 5.4 sont alors ajoutés avant d'irradier le milieu réactionnel dans un microondes 2 fois 30 minutes à 130°C. Le milieu est ensuite hydrolysé vers 5°C à l'aide de 5 mL d'eau et de 5 mL d'une solution aqueuse d'acide chlorhydrique (1N). Après l'hydrolyse, le milieu est dilué avec 50 mL d'eau et 50 mL de dichlorométhane puis filtré sur cartouche hydrophobe (Colonne d'extraction liquide/liquide 70 mL, Radleys®). La phase organique est récupérée et concentrée sous pression réduite après avoir ajouté 1,5 g de silice. Le résidu obtenu est chromatographié sur gel de silice en éluant avec un mélange de cyclohexane et d'acétate d'éthyle (6/4).

On obtient 0,112 g (72,3%) de composé attendu sous forme d'une poudre blanche.
Point de fusion (°C) : 179-181
LC-MS : M+H = 372
RMN ¹H (DMSO) δ (ppm): de 0,60 à 0,80 (m, 4H) ; 3,92 (m, 1H) ; 7,15 (s, 1H) ; de 7,30 à 7,39 (m, 3H) ; 7,61 (t, 1H) ; 7,88 (d, 1 H) ; 7,98 (d, 1H) ; 8,10 (m, 3H) ; 8,22 (s, 1H) ; 8,57 (m, 1H) ; 8,81 (d, 1H).

### Exemple 9: {3-[2-(4-Fluorophényl)pyrazolo[1,5-a]pyridin-5-yl]phényl}-(3-hydroxypyrrolidin-1-yl)méthanone (composé 22 du tableau)

0,100 g (0,30 mmol) d'acide 3-[2-(4-fluorophényl)pyrazolo[1,5-*a*]pyridin-5-yl]benzoïque préparés comme dans l'exemple 8 sont placés dans un ballon en présence de 0,170 mL (1,20 mmol) de triéthylamine et 20 mL de dichlorométhane. On ajoute alors 0,051 µL (0,39 mmol) de chlorformate d'isobutyle et on laisse agiter le milieu à température ambiante pendant 2 heures. 0,0341 g (0,39 mmol) de 3-hydroxypyrrolidine sont ajoutés au milieu et on laisse agiter à nouveau à température ambiante pendant 2 heures. Le milieu est ensuite dilué avec 50 mL d'eau et 50 mL de dichlorométhane. On filtre alors le milieu biphasique sur cartouche hydrophobe (Colonne d'extraction liquide/liquide 70 mL, Radleys®). La phase organique est récupérée et concentrée sous pression réduite après avoir ajouté 1 g de silice. Le résidu obtenu est chromatographié sur gel de silice en éluant avec un mélange de cyclohexane et d'acétate d'éthyle (6/4).

On obtient 0,063 g (50%) de composé attendu sous forme d'une cire jaune pâle.
Point de fusion (°C) : 173-175
LC-MS : M+H = 402
RMN ¹H (DMSO) δ (ppm): de 1,65 à 2,05 (m, 2H) ; de 3,40 à 3,70 (m, 4H) ; 4,32 (d, 1h) ; 5,00 (d, 1H) ; 7,11 (s, 1H) ; 7,32 (m, 3H) ; 7,60 (m, 2H) ; 7,93 (d, 2H) ; 8,08 (m, 3H) ; 8,77 (d, 1H).

### Exemple 10 : N-Méthyl-3-(2-p-tolylpyrazolo[1,5-a]pyridin-5-yl)benzamide (composé 328du tableau)

### 10.1 2-(4-Bromo-pyridin-2-yl)-1-p-tolyléthanone

Sous courant d'azote, 1 g (5,81 mmol) de 4-bromo-2-méthylpyridine et 1,75 g (11,60 mmol) de 4-méthylbenzoate de méthyle sont placés dans un ballon et dissous dans 30 mL de tétrahydrofurane anhydre. On refroidit à 5°C et on ajoute goutte à goutte 14 mL (14 mmol) d'une solution d'hexaméthyldisilazane de lithium (1M dans le tétrahydrofurane). Après addition, le mélange est agité à température ambiante pendant 2h30, puis refroidi à 5°C, avant d'ajouter progressivement 20 mL d'eau. Le milieu est ensuite dilué avec 200 mL d'acétate d'éthyle et 200 mL d'eau. La phase organique est séparée, séchée sur sulfate de sodium et filtrée. On ajoute ensuite au filtrat 5 g de silice avant le concentrer sous pression réduite. La poudre obtenue est utilisée comme dépôt solide pour une chromatographie sur gel de silice avec pour éluant un mélange de cyclohexane et d'acétate d'éthyle (95/5), on obtient 1,03 g (61%) de composé sous forme d'une poudre jaune.
LC-MS : M+H = 290

### 10.2 4-Bromo-2-(3-p-tolyl-2H-azirin-2-yl)pyridine

On place 1,03 g de 2-(4-bromo-pyridin-2-yl)-1-p-tolyléthanone obtenu à l'étape 13.1 dans un ballon avec 0,99 g (14,2 mmol) d'hydroxylamine monochlorhydrate, 3mL (37 mmol) de pyridine et 100 mL d'éthanol. On laisse agiter toute une nuit puis on concentre sous pression réduite le milieu réactionnel. Le résidu obtenu est alors repris avec 200 mL d'acétate d'éthyle et 200 mL d'eau. La phase organique est récupérée, séchée sur sulfate de sodium puis concentrée sous pression réduite. 1,10 g de composé sont récupérés et dissous dans un ballon contenant 0,660 mL (4,74 mmol) de triéthylamine et 30 mL de dichlorométhane. Le milieu réactionnel est ensuite refroidi vers 5°C puis 0,200 mL (1,42 mmol) d'anhydride de l'acide trifluoroacétique sont ajoutés goutte à goutte. Le milieu est ensuite agité à température ambiante pendant 3 heures avant d'être hydrolyse avec 100 mL d'eau. Le milieu est ensuite agité pendant 10 minutes avant d'être filtré sur cartouche hydrophobe (Colonne d'extraction liquide/liquide 70 mL, Radleys®). On ajoute ensuite au filtrat 1,2 g de silice avant de le concentrer sous pression réduite. La poudre obtenue est utilisée comme dépôt solide pour une chromatographie sur gel de silice avec pour éluant un mélange de cyclohexane et d'acétate d'éthyle (95/5). On récupère 0,746 g (77%) de composé attendu sous forme d'une poudre blanche.
RMN-¹H (DMSO) δ (ppm) : 2,42 (d, 3H) ; 3,45 (s, 1H) ; de 7,42 à 7,58 (m, 4H) ; 7,78 (m, 2H) ; 8,30 (d, 1 H).

### 10.3 5-Bromo-2-p-tolyl-pyrazolo[1,5-a]pyridine

0,746 g de 4-bromo-2-(3-p-tolyl-2H-azirin-2-yl)pyridine obtenue à l'étape 13.2 sont dissous en présence de 6,6 mg (0,052 mmol) de chlorure de fer (II) dans 30 mL de 1,2-diméthoxyéthane. Le milieu est alors porté à reflux pendant 6 heures. On rajoute ensuite 10 mg (0,078 mmol) de chlorure de fer (II) et on laisse ensuite agiter à nouveau à reflux pendant 3 heures. Le milieu est alors dilué avec 50 mL d'acétate d'éthyle et 50 mL d'eau. La phase organique est ensuite récupérée, séchée sur sulfate de sodium puis filtrée. On ajoute ensuite au filtrat 2 g de silice avant de le concentrer sous pression réduite. La poudre obtenue est utilisée comme dépôt solide pour une chromatographie sur gel de silice avec pour éluant un mélange de cyclohexane et d'acétate d'éthyle (85/15). On récupère 0,534g (71%) de composé attendu sous forme d'une poudre jaune.
LC-MS : M+H = 287
RMN-¹H (DMSO) δ (ppm) : 2,48 (m, 3H) ; 7,00 (m, 2H) ; 7,32 (m, 2H) ; 7,88 (m, 2H) ; 8,00 (m, 1 H) ; 8,68 (d, 1H).

### 10.4 N-méthyl-3-(4,4,5,5-tétraméthyl-[1,3,2]dioxaborolan-2-yl)benzamide

2,50 g (11,68 mmol) de 3-bromo-*N*-méthylbenzamide, 3,56 g (14,01 mmol) de bis(pinacolato)dibore, 3,43 g (35,04 mmol) d'acétate de potassium et 0,953 g (1,17 mmol) de [1,1'-bis(diphénylphosphino)ferrocène]dichloropalladium (II) sont mis en présence de 20 mL de dioxanne avant d'être irradiés par onde micro-onde à 130 °C pendant 45 minutes. Le milieu est ensuite dilué avec 150 mL d'acétate d'éthyle et 100 mL d'eau. La phase organique est récupérée et la phase aqueuse est extraite avec 2 fois 100 mL d'acétate d'éthyle. Les phases organiques sont alors réunies, séchées sur sulfate de sodium puis concentrées sous pression réduite après avoir ajouté 10 g de silice. Le résidu obtenu est chromatographié sur gel de silice en éluant avec un mélange de cyclohexane et d'acétate d'éthyle (8/2).

On obtient 1,39 g de composé attendu sous forme d'une poudre rosée (présence de pinacol). RMN ¹H (DMSO) δ (ppm): 1,30 (s, 12H) ; 2,78 (d, 3H) ; 7,48 (t, 1H) ; 7,80 (m, 1H) ; 7,95 (m, 1H) ; 8,12 (m, 1H) ; 8,50 (m, 1H).

### 10.5 N-Méthyl-3-(2-p-tolylpyrazolo[1,5-a]pyridin-5-yl)benzamide

On place 0,150g (0,52 mmol) de 5-bromo-2-p-tolylpyrazolo[1,5-*a*]pyridine obtenu à l'étape 13.3, 0,136 g (0,52 mmol) de *N*-méthyl-3-(4,4,5,5-tétraméthyl-[1,3,2]dioxaborolan-2-yl)benzamide obtenu à l'étape 13.4, 0,510 g (1,57 mmol) de carbonate de césium et 0,043 g (0,05 mmol) de [1,1'-bis(diphénylphosphino)ferrocène]dichloropalladium (II) dans 5 mL d'un mélange 9/1 de tétrahydrofurane et d'eau. Le milieu est agité à 65°C pendant 4 heures. Le milieu est ensuite dilué avec 50 mL de dichlorométhane et 50 mL d'eau. Le milieu biphasique est alors filtré sur cartouche hydrophobe (Colonne d'extraction liquide/liquide 70 mL, Radleys®). La phase organique est récupérée et concentrée sous pression réduite après avoir ajouté 1,5 g de silice. Le résidu obtenu est chromatographié sur gel de silice en éluant avec un mélange de cyclohexane et d'acétate d'éthyle (1/1).

On obtient 0,138 g (77,7%) de composé attendu sous forme d'une poudre beige.
Point de fusion (°C) : 204-206
LC-MS : M+H = 342
RMN ¹H (DMSO) δ (ppm): 2,38 (s, 3H) ; 2,85 (d, 3H) ; 7,10 (s, 1H) ; 7,31 (m, 3H) ; 7,62 (t, 1H) ; 7,90 (m, 3H) ; 7,98 (d, 1H) ; 8,08 (s, 1H) ; 8,29 (s, 1H) ; 8,61 (d, 1H) ; 8,81 (d, 1H).

### Exemple 11: 3-[3-Chloro-2-(4-fluorophényl)pyrazolo[1,5-a]pyridin-5-yl]-N-méthylbenzamide (exemple 34 du tableau)

0,100 g (0,29 mmol) de 3-[2-(4-fluorophényl)pyrazolo[1,5-*a*]pyridin-5-yl]-*N* méthylbenzamide obtenu selon le protocole 6 sont placés dans un ballon en présence de 3 mL de dichlorométhane. 0,060 g (0,45 mmol) de *N*-chlorosuccinimide sont ajoutés avant d'agiter le milieu à température ambiante pendant une nuit. Le milieu réactionnel est ensuite dilué avec 50 mL de dichlorométhane et 50 mL d'eau. On filtre alors le milieu biphasique sur cartouche hydrophobe (Colonne d'extraction liquide/liquide 70 mL, Radleys®). La phase organique est récupérée et concentrée sous pression réduite après avoir ajouté 1,2 g de silice. Le résidu obtenu est chromatographié sur gel de silice en éluant avec un mélange de cyclohexane et d'acétate d'éthyle (6/4).

On obtient 0,0592 g (53,8%) de composé attendu sous forme d'une poudre blanche.
Point de fusion (°C) : 221-223
LC-MS : M+H = 380
RMN-¹H (DMSO) δ (ppm) : 2,88 (d, 3H) ; 7,42 (m, 2H) ; 7,47 (dd, 1H) ; 7,64 (t, 1H) ; 7,93 (m, 1H) ; 8,01 (m, 1H) ; 8,05 (m, 1H) ; 8,10 (m, 2H) ; 8,31 (m, 1H) ; 8,63 (m, 1H) ; 8,88 (d, 1H).

Les tableaux qui suivent illustrent les structures chimiques de formule générale (I) (tableau 1) et les caractéristiques physicochimiques (tableau 2) de quelques exemples de composés selon l'invention.

Dans ces tableaux :
- la colonne « PF » renseigne les points de fusion des produits en degrés Celsius (°C) ;
- Me, Et représentent respectivement un groupe méthyle et éthyle ;
- * indique le ou les atome(s) de liaison.

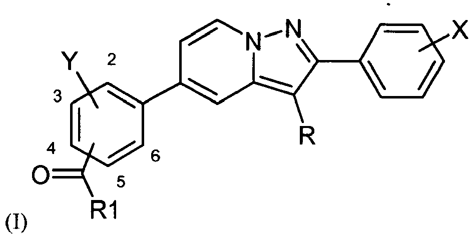

**Tableau 1**

| **N^{o}** | **R1** | **Position (C=O)R1** | **X** | **Y** | **R** |
|---|---|---|---|---|---|
| **1** | OMe | 3 | 4-Cl | H | H |
| | | | | | |
| **2** | NH₂ | 3 | 4-Cl | H | H |
| **3** | N(Me)₂ | 3 | 4-Cl | H | H |
| | | | | | |
| **4** | NHMe | 3 | 4-Cl | H | H |
| **5** | OMe | 3 | 4-F | H | H |
| | | | | | |
| **6** | NHMe | 3 | 4-F | H | H |
| **7** | 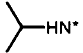 | 3 | 4-F | H | H |
| **8** | N(Me)₂ | 3 | 4-F | H | H |
| | | | | | |
| **9** | 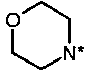 | 3 | 4-F | H | H |
| **10** | NHMe | 3 | 4-F | 2-Me | H |
| **11** | NHMe | 3 | 4-F | 4-Cl | H |
| **12** | NHMe | 3 | 4-F | 6-Cl | H |
| **13** | NHMe | 3 | 4-F | 6-Me | H |
| **14** | NHMe | 4 | 4-F | 3-F | H |
| **15** | 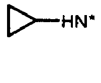 | 3 | 4-F | H | H |
| **16** | 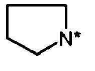 | 3 | 4-F | H | H |
| **17** | NHMe | 3 | 2,6-diF | H | H |
| **18** | NHMe | 3 | 2-F | H | H |
| **19** | NHMe | 3 | 4-CF₃ | H | H |
| **20** | NHMe | 4 | 4-F | H | H |
| **21** | NHMe | 2 | 4-F | H | H |
| **22** | 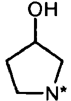 | 3 | 4-F | H | H |
| **23** | NHMe | 3 | 2,4-diF | H | H |
| **24** | NHMe | 3 | 4-OCF₃ | H | H |
| **25** | NHMe | 3 | 3,4-diF | H | H |
| **26** | NHMe | 3 | 3,5-diF | H | H |
| **27** | NHMe | 3 | 3-F | H | H |
| **28** | NHMe | 3 | 4-Me | H | H |
| **29** | NHMe | 3 | 4-OMe | H | H |
| **30** | NHMe | 3 | 3,4-diMe | H | H |
| **31** | NHMe | 3 | 4-CN | H | H |
| **32** | NHMe | 3 | 2,3-diF | H | H |
| **33** | NHMe | 3 | 2-Me | H | H |
| **34** | NHMe | 3 | 4-F | H | Cl |

**Tableau 2**

| **N^{o}** | **PF °C** | **RMN / [M+H]** |
|---|---|---|
| **1** | 180-182 | RMN-¹H (DMSO) δ (ppm) : 3,95 (s, 3H) ; 7,20 (s, 1H) ; 7,35 (d, 1H) ; 7,60 (d, 2H) ; 7,70 (t, 1H) ; de 8,00 à 8,20 (m, 5H) ; 8,35 (s, 1H) ; 8,85 (d, 1H). M+H = 363 |
| | | |
| **2** | 283-285 | RMN-¹H (DMSO) δ (ppm) : 7,17 (s, 1H) ; 7,36 (dd, 1H) ; 7,47 (s, 1H) ; 7,56 (m, 2H) ; 7,61 (t, 1H) ; 7,94 (m, 1H) ; 8,00 (m, 1H) ; 8,07 (m, 2H) ; 8,11 (m, 1H) ; 8,15 (s, 1H) ; 8,32 (m, 1H) ; 8,82 (d, 1H). M+H = 348 |
| **3** | 175-177 | RMN-¹H (DMSO) δ (ppm) : 3,02 (d, 6H) ; 7,15 (s, 1H) ; 7,35 (dd, 1H) ; 7,46 (m, 1H) ; de 7,50 à 7,67 (m, 3H) ; 7,85 (m, 1H) ; 7,91 (m, 1H) ; de 8,00 à 8,15 (m, 3H) ; 8,80 (d, 1H). M+H = 376 |
| **4** | 234-236 | RMN-¹H (DMSO) δ (ppm) : 2,85 (d, 3H) ; 7,18 (s, 1H) ; 7,35 (m, 1H) ; de 7,51 à 7,68 (m, 3H) ; 7,90 (m, 1H) ; 8,00 (m, 1H) ; de 8,02 à 8,12 (m, 3H) ; 8,28 (m, 1H) ; 8,62 (m, 1H) ; 8,82 (d, 1H). M+H = 362 |
| **5** | 162-164 | RMN-¹H (DMSO) δ (ppm) : 3,95 (s, 3H) ; 7,15 (s, 1H) ; de 7,30 à 7,38 (m, 3H) ; 7,70 (t, 1H) ; de 8,00 à 8,15 (m, 5H) ; 8,35 (m, 1H) ; 8,80 (d, 1 H). M+H = 347 |
| | | |
| **6** | 214-216 | RMN-¹H (DMSO) δ (ppm) : 2,85 (d, 3H) ; 7,15 (s, 1H) ; de 7,26 à 7,46 (m, 3H) ; 7,62 (m, 1H) ; 7,90 (m, 1H) ; 8,00 (m, 1H) ; de 8,05 à 8,21 (m, 3H) ; 8,29 (m, 1H) ; 8,60 (m, 1H) ; 8,82 (d, 1H). M+H = 346 |
| **7** | 210-212 | RMN ¹H (DMSO) δ (ppm): 1,23 (d, 6H) ; 4,18 (m, 1H) ; 7,14 (s, 1H) ; 7,34 (m, 3H) ; 7,62 (t, 1H) ; 7,90 (d, 1H) ; 7,99 (d, 1H) ; 8,07 (m, 3H) ; 8,24 (s, 1H) ; 8,47 (d, 1H) ; 8,80 (d, 1H). M+H=374. |
| **8** | 131-133 | RMN ¹H (DMSO) δ (ppm): 3,03 (d, 6H) ; 7,12 (s, 1H) ; de 7,30 à 7,39 (m, 3H) ; 7,47 (d, 1H) ; 7,57 (t, 1H) ; 7,82 (s, 1H) ; 7,90 (d, 1H) ; 8,07 (m, 3H) ; 8,78 (d, 1H).M+H=360. |
| | | |
| **9** | 152-154 | RMN ¹H (DMSO) δ (ppm): de 3,38 à 3,80 (m, 8H) ; 7,13 (s, 1H) ; 7,30 à 7,39 (m, 3H) ; 7,47 (d, 1H) ; 7,62 (t, 1H) ; 7,85 (s, 1H) ; 7,95 (d, 1H) ; 8,08 (m, 3H) ; 8,80 (d, 1H). M+H=402. |
| **10** | 240-242 | RMN ¹H (DMSO) δ (ppm): 2,37 (s, 3H) ; 2,80 (d, 3H) ; 6,86 (d, 1H) ; 7,10 (s, 1H) ; de 7,33 à 7,44 (m, 5H) ; 7,63 (s, 1H) ; 8,10 (m, 2H) ; 8,28 (s, 1H) ; 8,76 (d, 1H). M+H=360. |
| **11** | 243-245 | RMN ¹H (DMSO) δ (ppm): 2,83 (d, 3H) ; 7,12 (s, 1H) ; de 7,27 à 7,35 (m, 3H) ; 7,62 (d, 1H) ; 7,90 (m, 2H) ; de 8,04 à 8,16 (m, 3H) ; 8,46 (s, 1H) ; 8,80 (d, 1H). M+H=380. |
| **12** | 188-190 | RMN ¹H (DMSO) δ (ppm): 2,83 (d, 3H) ; 7,05 (d, 1H) ; 7,18 (s, 1H) ; 7,35 (m, 2H) ; 7,74 (d, 1H) ; 7,83 (s, 1H) ; 7,90 (d, 1H) ; 8,01 (s, 1H) ; 8,10 (m, 2H) ; 8,61 (s, 1H) ; 8,82 (d, 1H). M+H=380. |
| **13** | 185-187 | RMN ¹H (DMSO) δ (ppm): 2,49 (s, 3H) ; 2,83 (d, 3H) ; 6,97 (d, 1H) ; 7,10 (s, 1H) ; 7,35 (m, 2H) ; 7,46 (d, 1H) ; 7,70 (s, 1H) ; 7,83 (m, 2H) ; 8,08 (m, 2H) ; 8,47 (s, 1H) ; 8,77 (d, 1H). M+H=360. |
| **14** | 227-229 | RMN ¹H (DMSO) δ (ppm): 2,83 (d, 3H) ; 7,14 (s, 1H) ; de 7,30 à 7,39 (m, 3H) ; de 7,74 à 7,85 (m, 3H) ; 8,08 (m, 2H) ; 8,18 (s, 1H) ; 8,39 (s, 1H) ; 8,80 (d, 1H). M+H=364. |
| **15** | 179-181 | RMN 1H (DMSO) δ (ppm): de 0,60 à 0,80 (m, 4H) ; 3,92 (m, 1H) ; 7,15 (s, 1H) ; de 7,30 à 7,39 (m, 3H) ; 7,61 (t, 1H) ; 7,88 (d, 1H) ; 7,98 (d, 1H) ; 8,10 (m, 3H) ; 8,22 (s, 1H) ; 8,57 (m, 1H) ; 8,81 (d, 1H). M+H=372. |
| **16** | 164-166 | RMN ¹H (DMSO) δ (ppm): de 1,80 à 2,00 (m, 4H) ; de 3,43 à 3,56 (m, 4H) ; 7,12 (s, 1H) ; 7,30 à 7,39 (m, 3H) ; de 7,55 à 7,63 (m, 2H) ; 7,95 (m, 2H) ; 8,07 (m, 3H) ; 8,75 (d, 1H). M+H=386. |
| **17** | 207-209 | RMN ¹H (DMSO) δ (ppm): 2,85 (d, 3H) ; 7,00 (s, 1H) ; de 7,25 à 7,35 (m, 2H) ; 7,41 (d, 1H) ; de 7,54 à 7,68 (m, 2H) ; 7,93 (d, 1H) ; 8,00 (d, 1H) ; 8,17 (s, 1H) ; 8,28 (s, 1H) ; 8,60 (m, 1H) ; 8,92 (d, 1H). M+H=364. |
| **18** | 195-197 | RMN ¹H (DMSO) δ (ppm): 2,85 (d, 3H) ; 7,10 (s, 1H) ; de 7,32 à 7,43 (m, 3H) ; 7,48 (m, 1H) ; 7,65 (t, 1H) ; 7,91 (d, 1H) ; 7,99 (d, 1H) ; 8,18 (m, 2H) ; 8,28 (s, 1H) ; 8,60 (m, 1H) ; 8,87 (d, 1H). M+H=346. |
| **19** | 220-222 | RMN ¹H (DMSO) δ (ppm): 2,82 (d, 3H) ; 7,30 (s, 1H) ; 7,39 (d, 1H) ; 7,64 (t, 1H) ; de 7,84 à 7,95 (m, 3H); 8,00 (d, 1H) ; 8,13 (s, 1H) ; 8,27 (m, 3H) ; 8,60 (m, 1H) ; 8,85 (d, 1H). M+H=396. |
| **20** | 264-266 | RMN ¹H (DMSO) δ (ppm): 2,65 (d, 3H) ; 6,86 (d, 1H) ; 7,14 (s, 1H) ; 7,34 (m, 2H) ; de 7,48 à 7,59 (m, 4H) ; 7,66 (s, 1H) ; 8,09 (m, 2H) ; 8,23 (d, 1H) ; 8,72 (d, 1H). M+H=346. |
| **21** | 208-210 | RMN ¹H (DMSO) δ (ppm): 2,84 (d, 3H) ; 7,14 (s, 1H) ; 7,34 (m, 3H) ; 7,95 (m, 4H) ; 8,09 (m, 3H) ; 8,55 (d, 1H) ; 8,80 (d, 1H). M+H=346. |
| **22** | 173-175 | RMN ¹H (DMSO) δ (ppm): de 1,65 à 2,05 (m, 2H) ; de 3,40 à 3,70 (m, 4H) ; 4,32 (d, 1h) ; 5,00 (d, 1H) ; 7,11 (s, 1H) ; 7,32 (m, 3H) ; 7,60 (m, 2H) ; 7,93 (d, 2H) ; 8,08 (m, 3H) ; 8,77 (d, 1H). M+H=402. |
| **23** | 210-212 | RMN ¹H (DMSO) δ (ppm): 2,87 (d, 3H) ; 7,07 (s, 1H) ; 7,25 (t, 1H) ; de 7,38 à 7,48 (m, 2H) ; 7,63 (t, 1H) ; 7,91 (d, 1H) ; 7,98 (d, 1H) ; 8,18 (m, 2H) ; 8,28 (s, 1H) ; 8,60 (m, 1H) ; 8,87 (d, 1H). M+H=364. |
| **24** | 212-214 | RMN ¹H (DMSO) δ (ppm): 2,85 (d, 3H) ; 7,21 (s, 1H) ; 7,36 (d, 1H) ; 7,50 (d, 2H) ; 7,62 (t, 1H) ; 7,90 (d, 1H) ; 8,00 (d, 1H); 8,12 (s, 1H) ; 8,18 (d, 2H) ; 8,27 (s, 1H) ; 8,60 (d, 1H) ; 8,83 (d, 1H). MP=211°C. M+H=412. |
| **25** | 209-211 | RMN ¹H (DMSO) δ (ppm): 2,87 (d, 3H) ; 7,21 (s, 1H) ; 7,39 (d, 1H) ; de 7,54 à 7,68 (m, 2H) ; 7,90 (d, 2H) ; 8,00 (d, 1H) ; de 8,04 à 8,13 (m, 2H) ; 8,27 (s, 1H) ; 8,60 (d, 1H) ; 8,83 (d, 1H). M+H=364. |
| **26** | 230-232 | RMN ¹H (DMSO) δ (ppm): 2,85 (d, 3H) ; 7,30 (m, 2H) ; 7,39 (d, 1H) ; 7,63 (t, 1H) ; 7,78 (m, 2H) ; 7,92 (d, 1H) ; 8,01 (d, 1H); 8,13 (s, 1H) ; 8,27 (s, 1H) ; 8,61 (d, 1H) ; 8,84 (d, 1H). M+H=364. |
| **27** | 204-206 | RMN ¹H (DMSO) δ (ppm): 2,85 (d, 3H) ; 7,26 (m, 2H) ; 7,37 (d, 1H) ; de 7,53 à 7,64 (m, 2H) ; 7,82 (d, 1H) ; 7,90 (d, 2H) ; 8,00 (d, 1H) ; 8,11 (s, 1H) ; 8,27 (s, 1H) ; 8,61 (d, 1H) ; 8,85 (d, 1H). M+H=346. |
| **28** | 204-206 | RMN ¹H (DMSO) δ (ppm): 2,38 (s, 3H) ; 2,85 (d, 3H) ; 7,10 (s, 1H) ; 7,31 (m, 3H) ; 7,62 (t, 1H) ; 7,90 (m, 3H) ; 7,98 (d, 1H) ; 8,08 (s, 1H) ; 8,29 (s, 1H) ; 8,61 (d, 1H) ; 8,81 (d, 1H). M+H=342. |
| **29** | 211-213 | RMN ¹H (DMSO) δ (ppm): 2,85 (d, 3H) ; 3,82 (s, 3H) ; 7,06 (d, 3H) ; 7,31 (d, 1H) ; 7,64 (t, 1H) ; 7,90 (d, 1H) ; 7,98 (m, 3H) ; 8,07 (s, 1H) ; 8,27 (s, 1H) ; 8,61 (d, 1H) ; 8,80 (d, 1H). M+H=358. |
| **30** | 187-189 | RMN ¹H (DMSO) δ (ppm): 2,28 (s, 3H) ; 2,32 (s, 3H) ; 2,86 (d, 3H) ; 7,10 (s, 1H) ; 7,28 (d, 1H) ; 7,31 (d, 1H) ; 7,64 (t, 1H) ; 7,75 (d, 1H) ; 7,81 (s, 1H) ; 7,90 (d, 1H) ; 7,99 (d, 1H) ; 8,07 (s, 1H) ; 8,26 (s, 1H) ; 8,62 (d, 1H) ; 8,80 (d, 1H). M+H=356. |
| **31** | 252-254 | RMN ¹H (DMSO) δ (ppm): 2,87 (d, 3H) ; 7,32 (s, 1H) ; 7,43 (d, 1H) ; 7,63 (t, 1H) ; 7,92 (d, 1H) ; 8,00 (m, 3H) ; 8,15 (s, 1H) ; 8,27 (m, 3H) ; 8,62 (d, 1H) ; 8,87 (d, 1H). M+H=353. |
| **32** | 214-216 | RMN ¹H (DMSO) δ (ppm): 2,85 (d, 3H) ; 7,13 (s, 1H) ; de 7,32 à 7,43 (m, 2H) ; 7,50 (m, 1H) ; 7,63 (t, 1H) ; 7,90 (d, 1H) ; 7,97 (m, 2H) ; 8,17 (s, 1H) ; 8,27 (s, 1H) ; 8,60 (d, 1H) ; 8,88 (d, 1H). M+H=364. |
| **33** | 129-131 | RMN ¹H (DMSO) δ (ppm): 2,55 (s, 3H) ; 2,85 (d, 3H) ; 6,93 (s, 1H) ; 7,35 (m, 4H) ; 7,63 (t, 1H) ; 7,72 (m, 1H) ; 7,90 (d, 1H) ; 8,00 (d, 1H) ; 8,10 (s, 1H) ; 8,27 (s, 1H) ; 8,61 (d, 1H) ; 8,83 (d, 1H). M+H=342. |
| **34** | 221-223 | RMN-¹H (DMSO) δ (ppm) : 2,88 (d, 3H) ; 7,42 (m, 2H) ; 7,47 (dd, 1H) ; 7,64 (t, 1H) ; 7,93 (m, 1H) ; 8,01 (m, 1H) ; 8,05 (m, 1H) ; 8,10 (m, 2H) ; 8,31 (m, 1H) ; 8,63 (m, 1H) ; 8,88 (d, 1H). M+H = 380 |

Les composés selon l'invention ont fait l'objet d'essais pharmacologiques permettant de déterminer leur effet modulateur sur NOT.

### Evaluation de l'activité in vitro sur cellules N2A

L'activité des composés selon l'invention a été évaluée sur une lignée cellulaire (N2A) exprimant de manière endogène le récepteur de souris Nurr1 et transfectée de manière stable avec l'élément de réponse liant NOT (NBRE) couplé au gène rapporteur luciférase. Les essais ont été réalisés selon le mode opératoire décrit ci dessous.

La lignée cellulaire Neuro-2A provient de source commerciale standard (ATCC). Le clone Neuro-2A a été obtenu à partir d'une tumeur spontanée provenant d'une souche de souris A albino par R.J Klebe et col. Cette lignée Neuro-2A est ensuite stablement transfectée avec 8NBRE-luciférase. Les cellules N2A-8NBRE sont cultivées jusqu'à confluence dans des flacons de culture de 75 cm² contenant du DMEM supplémenté par 10% de sérum foetal de veau, 4,5 g/L de glucose et 0,4 mg/ml de Généticine. Après une semaine de culture, les cellules sont récupérées par de la trypsine 0,25% pendant 30 secondes puis remises en suspension dans du DMEM sans rouge de phénol contenant 4,5g/L de glucose, 10% de sérum délipidé Hyclone et déposées dans des plaques blanches 96 puits à fond transparent. Les cellules sont déposées à raison de 60.000 par puits dans 75 µL pendant 24 heures avant l'addition des produits. Les produits sont appliqués dans 25µL et incubés 24 heures supplémentaires. Le jour de la mesure, on ajoute à chaque puits un volume équivalent (100µL) de Steadylite, puis on attend 30 minutes pour obtenir une lyse complète des cellules et la production maximale du signal. Les plaques sont ensuite mesurées dans un compteur de luminescence pour microplaques après avoir été scellées par un film adhésif. Les produits sont préparés sous forme de solution stock à 10⁻² M, puis dilués dans 100% de DMSO. Chaque concentration de produit est préalablement diluée dans du milieu de culture avant incubation avec les cellules contenant ainsi 0,625% final de DMSO.

Les meilleurs composés ont une CE50 comprise entre 0,1 nM et 10 µM.

Par exemple, les composés n°2, 4, 10, 14, 16 et 26 ont montré une CE50 de 45 ; 2 ; 6,6 ; 125 ; 326 et 1,3 nM respectivement.

Il apparaît donc que les composés selon l'invention ont un effet modulateur de NOT.

Les composés selon l'invention peuvent donc être utilisés pour la préparation de médicaments pour leur application en thérapeutique dans le traitement ou la prévention de maladies impliquant les récepteurs NOT.

Ainsi, selon un autre de ses aspects, l'invention a pour objet des médicaments qui comprennent un composé de formule (I), ou un sel d'addition de ce dernier à un acide pharmaceutiquement acceptable.

Ces médicaments trouvent leur emploi en thérapeutique, notamment dans le traitement et la prévention des maladies neurodégénératives telles que par exemple la maladie de Parkinson, d'Alzheimer, les tauopathies (ex. la paralysie progressive supranucléaire, la démence fronto-temporale, la dégénérescence corticobasale, la maladie de Pick); les traumatismes cérébraux comme l'ischémie et les traumatismes crâniens et l'épilepsie ; les maladies psychiatriques comme la schizophrénie, la dépression, la dépendance à une substance, les troubles du déficit de l'attention et de l'hyperactivité ; les maladies inflammatoires du système nerveux central comme la sclérose en plaque, encéphalite, myélite et encéphalomyélite et autres maladies inflammatoires comme les pathologies vasculaires, l'athérosclérose, les inflammations des articulations, l'arthrose, l'arthrite rhumatoïde ; l'ostéoarthrite, la maladie de Crohn, colite ulcéreuse; les maladies inflammatoires allergiques telle que l'asthme, les maladies auto-immunes comme le diabète de type 1, lupus, sclérodermies, Syndrome de Guillain-Barré, maladie d'Addison et autres maladies immuno-médiées; l'ostéoporose; les cancers.

Ces composés pourraient être aussi utilisés comme traitement associé à des greffes et/ou transplantations de cellules souches.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, en peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscarmellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention divulgue également une méthode de traitement des pathologies ci-dessus indiquées qui comprend l'administration, à un patient, d'une dose efficace d'un composé selon l'invention, ou un de ses sels pharmaceutiquement acceptables.

## Revendications

1. Composés de formule (I) : 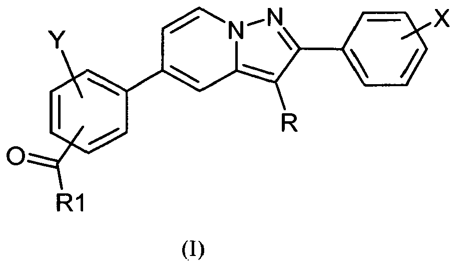 dans laquelle :
R représente un atome d'hydrogène ou d'halogène ou un groupe (C1-C6)alkyle;
X représente un ou plusieurs substituants choisis parmi un atome d'hydrogène ou d'halogène, un groupe (C1-C6)alkyle, halogéno(C1-C6)alkyle, (C1-C6)alcoxy, halogéno(C1-C6)alcoxy, cyano, hydroxy ou hydroxy(C1-C6)alkyle;
Y représente un atome d'hydrogène, d'halogène ou un groupe (C1-C6)alkyle;
R1 représente un groupe NR2R3 ou OR4;
R2 et R3 représentent, indépendamment l'un de l'autre, un atome d'hydrogène, un groupe (C1-C6)alkyle, hydroxy(C1-C6)alkyle ou oxo(C1-C6)alkyle, ou bien R2 et R3 forment avec l'atome d'azote qui les porte un hétérocycle éventuellement substitué par un groupe(C1-C6)alkyle, hydroxy ou oxo,
R4 représente un groupe (C1-C6)alkyle, hydroxy(C1-C6)alkyle ou oxo(C1-C6)alkyle, à l'état de base ou de sel d'addition à un acide.

2. Composés de formule (I) selon la revendication 1, **caractérisés en ce que**
R représente un atome d'hydrogène ou de chlore,
X représente un ou plusieurs substituants choisis parmi un atome d'halogène, un groupe (C1-C6)alkyle, halogéno(C1-C6)alkyle, (C1-C6)alcoxy, halogéno(C1-C6)alcoxy, ou cyano,
Y représente un atome d'hydrogène, un atome d'halogène ou un groupe (C1-C6)alkyle;
R1 représente un groupe OR4,
R4 représente un groupe méthyle, à l'état de base ou de sel d'addition à un acide.

3. Composés de formule (I) selon la revendication 1, **caractérisé en ce que**
R représente un atome d'hydrogène ou de chlore,
X représente un ou plusieurs substituants choisis parmi un atome de chlore ou de fluor, un groupe méthyle, trifluorométhyle, méthoxy, trifluorométhoxy ou cyano,
Y représente un atome d'hydrogène, de chlore, de fluor ou un groupe méthyle;
R1 représente un groupe OR4,
R4 représente un groupe méthyle, à l'état de base ou de sel d'addition à un acide.

4. Composés de formule (I) selon la revendication 1, **caractérisés en ce que**
R représente un atome d'hydrogène ou de chlore ;
X représente un ou plusieurs substituants choisis parmi un un atome d'halogène, un groupe (C1-C6)alkyle, halogéno(C1-C6)alkyle, (C1-C6)alcoxy, halogéno(C1-C6)alcoxy, ou cyano;
Y représente un atome d'hydrogène, un atome d'halogène ou un groupe (C1-C6)alkyle;
R1 représente un groupe NR2R3,
R2 et R3 représentent, indépendamment l'un de l'autre, un atome d'hydrogène, un groupe méthyle, éthyle, isopropyle, cyclopropyle, ou bien R2 et R3 forment avec l'atome d'azote qui les porte un groupe morpholinyle ou pyrrolidine éventuellement substitué par un groupe hydroxy, à l'état de base ou de sel d'addition à un acide.

5. Composés de formule (I) selon la revendication 1, **caractérisés en ce que**
R représente un atome d'hydrogène ou de chlore,
X représente un ou plusieurs substituants choisis parmi un atome de chlore ou de fluor, un groupe méthyle, trifluorométhyle, méthoxy, trifluomométhoxy ou cyano,
Y représente un atome d'hydrogène ou de chlore ou un groupe méthyle;
R1 représente un groupe NR2R3,
R2 et R3 représentent, indépendamment l'un de l'autre, un atome d'hydrogène, un groupe méthyle, éthyle, isopropyle, cyclopropyle, ou bien R2 et R3 forment avec l'atome d'azote qui les porte un groupe morpholinyle ou pyrrolidine éventuellement substitué par un groupe hydroxy, à l'état de base ou de sel d'addition à un acide.

6. Composés de formule (I) selon l'une quelconque des revendications 1 à 5 :
• 3-[2-(4-Chlororophényl)pyrazolo[1,5-*a*]pyridin-5-yl]benzoate de methyle
• 3-[2-(4-chlorophényl)pyrazolo[1,5-*a*]pyridin-5-y]benzamide
• 3-[2-(4-Chlorophényl)pyrazolo[1,5-*a*]pyridin-5-yl]-*N*,*N*-diméthylbezamide
• 3-[2-(4-Chlorophényl)pyrazolo[1,5-*a*]pyridin-5-yl]-*N*-méthylbenzamide
• 3-[2-(4-Fluorophényl)pyrazolo[1,5-*a*]pyridin-5-yl]benzoate de méthyle,
• 3-[2-(4-Fluorophényl)pyrazolo[1,5-*a*]pyridin-5-yl]-*N*-méthylbenzamide
• 3-[2-(4-Fluorophényl)pyrazolo[1,5-*a*]pyridin-5-yl]-*N*-isopropylbenzamide
• 3-[2-(4-Fluorophényl)pyrazolo[1,5-*a*]pyridin-5-yl]-*N*,*N*-diméthylbenzamide
• {3-[2-(4-Fluorophényl)pyrazolo[1,5-*a*]pyridin-5-yl]phényl}morpholin-4-yl-méthanone
• 3-[2-(4-Fluorophényl)pyrazolo[1,5-*a*]pyridin-5-yl]-2,*N*-diméthylbenzamide
• 2-Chloro-5-[2-(4-fluorophényl)pyrazolo[1,5-*a*]pyridin-5-yl]-*N-*méthylbenzamide
• 4-Chloro-3-[2-(4-fluorophényl)pyrazolo[1,5-*a*]pyridin-5-yl]-*N*-méthylbenzamide
• 3-[2-(4-Fluorophényl)pyrazolo[1,5-*a*]pyridin-5-yl]-4,*N*-diméthylbenzamide
• 2-Fluoro-4-[2-(4-fluorophényl)pyrazolo[1,5-*a*]pyridin-5-yl]-*N*-méthylbenzamide
• *N*-Cyclopropyl-3-[2-(4-fluorophényl)pyrazolo[1,5-*a*]pyridin-5-yl]benzamide
• {3-[2-(4-Fluorophényl)pyrazolo[1,5-*a*]pyridin-5-yl]phényl}pyrrolidin-1-ylméthanone
• 3-[2-(2,6-Difluorophényl)pyrazolo[1,5-*a*]pyridin-5-yl-méthylbenzamide
• 3-[2-(2-Fluorophényl)pyrazolo[1,5-*a*]pyridin-5-yl]-*N*-méthylbenzamide
• N-Méthyl-3-[2-(4-trifluorométhylphényl)pyrazolo[1,5-*a*]pyridin-5-yl]benzamide
• 4-[2-(4-Fluorophényl)pyrazolo[1,5-*a*]pyridin-5-yl]-*N*-méthylbenzamide
• 2-[2-(4-Fluorophényl)pyrazolo[1,5-*a*]pyridin-5-yl]-*N*-méthylbenzamide
• {3-[2-(4-Fluorophényl)pyrazolo[1,5-*a*]pyridin-5-yl]phényl}-(3-hydroxypyrrolidin-1-yl)méthanone
• 3-[2-(2,4-Difluorophényl)pyrazolo[1,5-*a*]pyridin-5-yl]-*N*-méthylbenzamide
• *N*-Méthyl-3-[2-(4-trifluorométhoxyphényl)pyrazolo[1,5-*a*]pyridin-5-yl]benzamide
• 3-[2-(3,4-Difluorophényl)pyrazolo[1,5-*a*]pyridin-5-yl]-*N*-méthylbenzamide
• 3-[2-(3,5-Difluorophényl)pyrazolo[1,5-*a*]pyridin-5-yl]-*N*-méthylbenzamide
• 3-[2-(3-Fluorophényl)pyrazolo[1,5-*a*]pyridin-5-yl]-*N*-méthylbenzamide
• *N*-Méthyl-3-(2-p-tolylpyrazolo[1,5-*a*]pyridin-5-yl)benzamide
• 3-[2-(4-Méthoxyphényl)pyrazolo[1,5-*a*]pyridin-5-yl]-*N*-méthylbenzamide
• 3-[2-(3,4-Diméthylphényl)pyrazolo[1,5-*a*]pyridin-5-yl]-*N*-méthylbenzamide
• 3-[2-(4-Cyanophényl)pyrazolo[1,5-*a*]pyridin-5-yl]-*N*-méthylbenzamide
• 3-[2-(2,3-Difluorophényl)pyrazolo[1,5-a]pyridin-5-yl]-*N*-méthylbenzamide
• *N*-Méthyl-3-(2-*o*-tolylpyrazolo[1,5-*a*]pyridin-5-yl)benzamide
• 3-[3-Chloro-2-(4-fluorophényl)pyrazolo[1,5-*a*]pyridin-5-yl]-*N*-méthylbenzamide

7. Médicament, **caractérisé en ce qu'**il comprend un composé de formule (I) selon l'une quelconque des revendications 1 à 6, ou un sel d'addition de ce composé à un acide pharmaceutiquement acceptable.

8. Composition pharmaceutique, **caractérisée en ce qu'**elle comprend un composé de formule (I) selon l'une quelconque des revendications 1 à 6, ou un sel pharmaceutiquement acceptable, de ce composé, ainsi qu'au moins un excipient pharmaceutiquement acceptable.

9. Utilisation d'un composé de formule (I) selon l'une quelconque des revendications 1 à 6 pour la préparation d'un médicament destiné au traitement et à la prévention des maladies neurodégénératives.

10. Utilisation d'un composé de formule (I) selon l'une quelconque des revendications 1 à 6 pour la préparation d'un médicament destiné au traitement et à la prévention des traumatismes cérébraux et de l'épilepsie.

11. Utilisation d'un composé de formule (I) selon l'une quelconque des revendications 1 à 6 pour la préparation d'un médicament destiné au traitement et à la prévention des maladies psychiatriques.

12. Utilisation d'un composé de formule (1) selon l'une quelconque des revendications 1 à 6 pour la préparation d'un médicament destiné au traitement et à la prévention des maladies inflammatoires.

13. Utilisation d'un composé de formule (I) selon l'une quelconque des revendications 1 à 6 pour la préparation d'un médicament destiné au traitement et à la prévention de l'ostéoporose et les cancers.

14. Utilisation d'un composé de formule (I) selon l'une quelconque des revendications 1 à 6 pour la préparation d'un médicament destiné au traitement et à la prévention de la maladie de Parkinson, d'Alzheimer, des tauopathies, de la sclérose en plaque.

15. Utilisation d'un composé de formule (I) selon l'une quelconque des revendications 1 à 6 pour la préparation d'un médicament destiné au traitement et à la prévention de la schizophrénie, la dépression, la dépendance à une substance, les troubles du déficit de l'attention et de l'hyperactivité.

16. Composé intermédiaire de formule (VI-1)

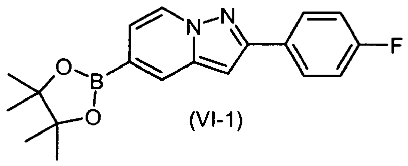

17. Utilisation du composé selon la revendication 16 pour la synthèse de produits de formule générale (I) selon la revendication 1.

## Patentansprüche

1. Verbindungen der Formel (I): 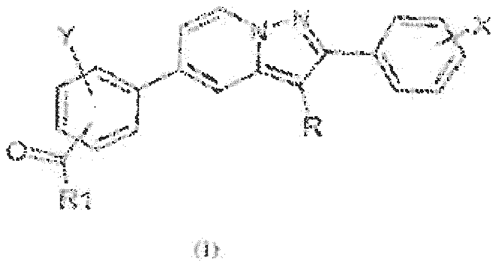 worin:
R für ein Wasserstoff- oder Halogenatom oder eine (C1-C6)-Alkylgruppe steht;
X für einen oder mehrere Substituenten steht, die aus einem Wasserstoff- oder Halogenatom und einer (C1-C6)-Alkyl-, Halogen-(C1-C6)-alkyl-, (C1-C6)-Alkoxy-, Halogen-(C1-C6)-alkoxy, Cyano-, Hydroxy-oder Hydroxy-(C1-C6)-alkylgruppe ausgewählt sind;
Y für ein Wasserstoff- oder Halogenatom oder eine (C1-C6)-Alkylgruppe steht;
R1 für eine NR2R3- oder OR4-Gruppe steht;
R2 und R3 unabhängig voneinander für ein Wasserstoffatom oder eine (C1-C6)-Alkyl-, Hydroxy-(C1-C6)-Alkyl- oder Oxo-(C1-C6)-alkylgruppe stehen oder R2 und R3 mit dem Stickstoffatom, das sie trägt, einen gegebenenfalls durch eine (C1-C6)-Alkyl-, Hydroxy- oder Oxogruppe substituierten Heterocyclus bilden,
R4 für eine (C1-C6)-Alkyl-, Hydroxy-(C1-C6)-Alkyl- oder Oxo-(C1-C6)-alkylgruppe steht, in Basen- oder Säureadditionssalzform.

2. Verbindungen der Formel (I) nach Anspruch 1, **dadurch gekennzeichnet, dass**
R für ein Wasserstoff- oder Chloratom steht,
X für einen oder mehrere Substituenten steht, die aus einem Halogenatom und einer (C1-C6)-Alkyl-, Halogen-(C1-C6)-alkyl-, (C1-C6)-Alkoxy-, Halogen-(C1-C6)-alkoxy- oder Cyanogruppe ausgewählt sind;
Y für ein Wasserstoffatom, ein Halogenatom oder eine (C1-C6)-Alkylgruppe steht;
R1 für eine OR4-Gruppe steht;
R4 für eine Methylgruppe steht, in Basen- oder Säureadditionssalzform.

3. Verbindungen der Formel (I) nach Anspruch 1, **dadurch gekennzeichnet, dass**
R für ein Wasserstoff- oder Chloratom steht,
X für einen oder mehrere Substituenten steht, die aus einem Chlor- oder Fluoratom und einer Methyl-, Trifluormethyl-, Methoxy-, Trifluormethoxy- oder Cyanogruppe ausgewählt sind;
Y für ein Wasserstoff-, Chlor- oder Fluoratom oder eine Methylgruppe steht;
R1 für eine OR4-Gruppe steht;
R4 für eine Methylgruppe steht, in Basen- oder Säureadditionssalzform.

4. Verbindungen der Formel (I) nach Anspruch 1, **dadurch gekennzeichnet, dass**
R für ein Wasserstoff- oder Chloratom steht,
X für einen oder mehrere Substituenten steht, die aus einem Halogenatom und einer (C1-C5)-Alkyl-, Halogen-(C1-C6)-alkyl-, (C1-C6)-Alkoxy-, Halogen-(C1-C6)-alkoxy- oder Cyanogruppe ausgewählt sind;
Y für ein Wasserstoffatom, ein Halogenatom oder eine (C1-C6)-Alkylgruppe steht;
R1 für eine NR2R3-Gruppe steht;
R2 und R3 unabhängig voneinander für ein Wasserstoffatom oder eine Methyl-, Ethyl-, Isopropyl- oder Cyclopropylgruppe stehen oder R2 und R3 mit dem Stickstoffatom, das sie trägt, eine gegebenenfalls durch eine Hydroxygruppe substituierte Morpholinyl- oder Pyrrolidingruppe bilden, in Basen- oder Säureadditionssalzform.

5. Verbindungen der Formel (I) nach Anspruch 1, **dadurch gekennzeichnet, dass**
R für ein Wasserstoff- oder Chloratom steht, 1
X für einen oder mehrere Substituenten steht, 1 die aus einem Chlor- oder Fluoratom und einer Methyl-, Trifluormethyl-, Methoxy-, Trifluormethoxy- oder Cyanogruppe ausgewählt sind;
Y für ein Wasserstoff- oder Chloratom oder eine Methylgruppe steht;
R1 für eine NR2R3-Gruppe steht;
R2 und R3 unabhängig voneinander für ein Wasserstoffatom oder eine Methyl-, Ethyl-, Isopropyl- oder Cyclopropylgruppe stehen oder R2 und R3 mit dem Stickstoffatom, das sie trägt, eine gegebenenfalls durch eine Hydroxygruppe substituierte Morpholinyl- oder Pyrrolidingruppe bilden,
in Basen- oder Säureadditionssalzform.

6. Verbindungen der Formel (I) nach einem der Ansprüche 1 bis 5:
• 3-[2-(4-Chlorphenyl)pyrazolo[1,5-a]pyridin-5-yl]benzoesäuremethylester
• 3-[2-(4-Chlophenyl)pyrazolo[1,5-a]pyridin-5-yl]benzamid
• 3-[2-(4-Chlorphenyl)pyrazolo[1,5-a]pyridin-5-yl]-N,*N*-dimethylbenzamid
• 3-[2-(4-Chlorphenyl)pyrazolo[1,5-a]pyridin-5-yl]-*N*-methylbenzamid
• 3-[2-(4-Fluorphenyl)pyrazolo[1,5-a]pyridin-5-yl]benzoesäuremethylester
• 3-[2-(4-Fluorphenyl)pyrazolo[1,5-a]pyridin-5-yl]-*N*-methylbenzamid
• 3-[2-(4-Fluorphenyl)pyrazolo[1,5-a]pyridin-5-yl]-*N*-isopropylbenzamid
• 3-[2-(4-Fluorphenyl)pyrazolo[1,5-a]pyridin-5-yl]-N,*N*-dimethylbenzamid
• {3-[2-(4-Fluorphenyl)pyrazolo[1,5-a]pyridin-5-yl]phenyl}morpholin-4-yl-methanon
• 3-[2-(4-Fluorphenyl)pyrazolo[1,5-a]pyridin-5-yl]-2,*N*-dimethylbenzamid
• 2-Chlor-5-[2-(4-fluorphenyl)pyrazolo[1,5-a]-pyridin-5-yl]-*N*-methylbenzamid
• 4-Chlor-3-[2-(4-fluorphenyl)pyrazolo[1,-5-a]-pyridin-5-yl]-*N*-methylbenzamid
• 3-[2-(4-Fluorphenyl)pyrazolo[1,5-a]pyridin-5-yl]-4,*N*-dimethylbenzamid
• 2-Fluor-4-[2-(4-fluorphenyl)pyrazolo[1,5-a]-pyridin-5-yl]-*N*-methylbenzamid
• *N*-Cyclopropyl-3-[2-(4-fluorphenyl)pyrazolo[1,5-a]pyridin-5-y1]benzamid
• {3-[2-(4-Fluorphenyl)pyrazolo[1,5-a]pyridin-5-yl]phenyl}pyrrolidin-1-ylmethanon
• 3-[2-(2,6-Difluorphenyl)pyrazolo[1,5-a]pyridin-5-yl-methylbenzamid
• 3-[2-(2-Fluorphenyl)pyrazolo[1,5-a]pyridin-5-yl]-*N*-methylbenzamid
• N-Methyl-3-[2-(4-trifluormethylphenyl)pyrazolo-[1,5-a] pyridin-5-yl]benzamid
• 4-[2-(4-Fluorphenyl)pyrazolo[1,5-a]pyridin-5-yl]-*N*-methylbenzamid
• 2-[2-(4-Fluorphenyl)pyrazolo[1,5-a]pyridin-5-yl]-*N*-methylbenzamid
• {3-[2-(4-Fluorphenyl)pyrazolo[1,5-a]pyridin-5-yl]phenyl}-(3-hydroxypyrrolidin-1-yl)methanon
• 3-[2-(2,4-Difluorphenyl)pyrazolo[1,5-a]pyridin-5-yl]-*N*-methylbenzamid
• *N*-Methyl-3-[2-(4-trifluormethoxyphenyl)-pyrazolo[1,5-a]pyridin-5-yl]benzamid
• 3-[2-(3,4-Difluorphenyl)pyrazolo[1,5-a]pyridin-5-yl]-*N*-methylbenzamid
• 3-[2-(3,5-Difluorphenyl)pyrazolo[1,5-a]pyridin-5-yl]-*N*-methylbenzamid
• 3-[2-(3-Fluorphenyl)pyrazolo[1,5-a]pyridin-5-yl]-*N*-methylbenzamid
• *N*-Methyl-3-(2-p-tolylpyrazolo[1,5-a]pyridin-5-yl)benzamid
• 3-[2-(4-Methoxyphenyl)pyrazolo[1,5-a]pyridin-5-yl]-*N*-methylbenzamid
• 3-[2-(3,4-Dimethylphenyl)pyrazolo[1,5-a]-pyridin-5-yl]-*N*-methylbenzamid
• 3-[2-(4-Cyanophenyl)pyrazolo[1,5-a]pyridin-5-yl]-*N*-methylbenzamid
• 3-[2-(2,3-Difluorphenyl)pyrazolo[1,5-a]pyridin-5-yl]-*N*-methylbenzamid
• *N*-Methyl-3-(2-o-tolylpyrazolo[1,5-a]pyridin-5-yl)benzamid
• 3-[3-Chlor-2-(4-fluorphenyl)pyrazolo[1,5-a]-pyridin-5-yl]-*N*-methylbenzamid.

7. Medikament, **dadurch gekennzeichnet, dass** es eine Verbindung der Formel (I) nach einem der Ansprüche 1 bis 6 oder ein Additionssalz dieser Verbindung mit einer pharmazeutisch unbedenklichen Säure umfasst.

8. Pharmazeutische Zusammensetzung, **dadurch gekennzeichnet, dass** sie eine Verbindung der Formel (I) nach einem der Ansprüche 1 bis 6 oder ein pharmazeutisch unbedenkliches Salz dieser Verbindung sowie mindestens einen pharmazeutisch unbedenklichen Hilfsstoff umfasst.

9. Verwendung einer Verbindung der Formel (I) nach einem der Ansprüche 1 bis 6 zur Herstellung eines Medikaments zur Behandlung oder Prävention von neurodegenerativen Erkrankungen.

10. Verwendung einer Verbindung der Formel (I) nach einem der Ansprüche 1 bis 6 zur Herstellung eines Medikaments zur Behandlung oder Prävention von Hirntraumen und Epilepsie.

11. Verwendung einer Verbindung der Formel (I) nach einem der Ansprüche 1 bis 6 zur Herstellung eines Medikaments zur Behandlung oder Prävention von psychiatrischen Erkrankungen.

12. Verwendung einer Verbindung der Formel (I) nach einem der Ansprüche 1 bis 6 zur Herstellung eines Medikaments zur Behandlung oder Prävention von entzündlichen Erkrankungen.

13. Verwendung einer Verbindung der Formel (I) nach einem der Ansprüche 1 bis 6 zur Herstellung eines Medikaments zur Behandlung oder Prävention von Osteoporose und Krebserkrankungen.

14. Verwendung einer Verbindung der Formel (I) nach einem der Ansprüche 1 bis 6 zur Herstellung eines Medikaments zur Behandlung oder Prävention von Parkinson-Krankheit, Alzheimer-Krankheit, Tauopathien und Multipler Sklerose.

15. Verwendung einer Verbindung der Formel (I) nach einem der Ansprüche 1 bis 6 zur Herstellung eines Medikaments zur Behandlung oder Prävention von Schizophrenie, Depression, Substanzabhängigkeit und Aufmerksamkeitsdefizit-Hyperaktivitätssyndrom.

16. Zwischenverbindung der Formel (VI-1)

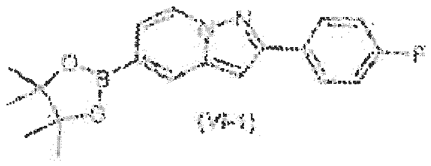

17. Verwendung der Verbindung nach Anspruch 16 zur Synthese von Produkten der allgemeinen Formel (I) nach Anspruch 1.

## Claims

1. Compounds of formula (I): 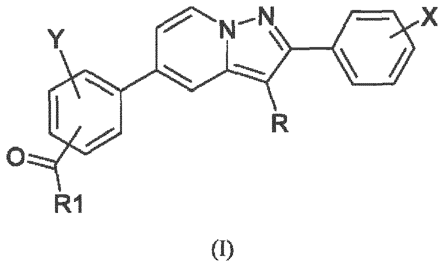 in which:
R represents a hydrogen or halogen atom or a group (C1-C6)alkyl;
X represents one or more substituents chosen from a hydrogen or halogen atom and a group (C1-C6)alkyl, halo(C1-C6)alkyl, (C1-C6)alkoxy, halo(C1-C6)alkoxy, cyano, hydroxyl or hydroxy(C1-C6)alkyl;
Y represents a hydrogen or halogen atom or a group (C1-C6)alkyl;
R1 represents a group NR2R3 or OR4;
R2 and R3 represent, independently of each other, a hydrogen atom or a group (C1-C6)alkyl, hydroxy(C1-C6)alkyl or oxo(C1-C6)alkyl, or alternatively R2 and R3 form, with the nitrogen atom that bears them, a heterocycle optionally substituted with a group (C1-C6)alkyl, hydroxyl or oxo,
R4 represents a group (C1-C6)alkyl, hydroxy(C1-C6)alkyl or oxo(C1-C6)alkyl, in the form of base or of acid-addition salt.

2. Compounds of formula (I) according to Claim 1, **characterized in that**
R represents a hydrogen or chlorine atom,
X represents one or more substituents chosen from a halogen atom and a group (C1-C6)alkyl, halo(C1-C6)alkyl, (C1-C6)alkoxy, halo(C1-C6)alkoxy or cyano,
Y represents a hydrogen atom, a halogen atom or a group (C1-C6)alkyl;
R1 represents a group OR4,
R4 represents a methyl group, in the form of base or of acid-addition salt.

3. Compounds of formula (I) according to Claim 1, **characterized in that**
R represents a hydrogen or chlorine atom,
X represents one or more substituents chosen from a chlorine or fluorine atom and a methyl, trifluoromethyl, methoxy, trifluoromethoxy or cyano group,
Y represents a hydrogen, chlorine or fluorine atom or a methyl group,
R1 represents a group OR4,
R4 represents a methyl group, in the form of base or of acid-addition salt.

4. Compounds of formula (I) according to Claim 1, **characterized in that**
R represents a hydrogen or chlorine atom,
X represents one or more substituents chosen from a halogen atom and a group (C1-C6)alkyl, halo(C1-C6)alkyl, (C1-C6)alkoxy, halo(C1-C6)alkoxy or cyano,
Y represents a hydrogen atom, a halogen atom or a group (C1-C6)alkyl;
R1 represents a group NR2R3,
R2 and R3 represent, independently of each other, a hydrogen atom or a methyl, ethyl, isopropyl or cyclopropyl group, or alternatively R2 and R3 form, with the nitrogen atom that bears them, a morpholinyl or pyrrolidinyl group optionally substituted with a hydroxyl group, in the form of base or of acid-addition salt.

5. Compounds of formula (I) according to Claim 1, **characterized in that**
R represents a hydrogen or chlorine atom,
X represents one or more substituents chosen from a chlorine or fluorine atom and a methyl, trifluoromethyl, methoxy, trifluoromethoxy or cyano group,
Y represents a hydrogen or chlorine atom or a methyl group,
R1 represents a group NR2R3,
R2 and R3 represent, independently of each other, a hydrogen atom or a methyl, ethyl, isopropyl or cyclopropyl group, or alternatively R2 and R3 form, with the nitrogen atom that bears them, a morpholinyl or pyrrolidinyl group optionally substituted with a hydroxyl group, in the form of base or of acid-addition salt.

6. Compounds of formula (I) according to any one of Claims 1 to 5:
• Methyl 3-[2-(4-chlorophenyl)pyrazolo[1,5-*a*]pyridin-5-yl]benzoate
• 3-[2-(4-Chlorophenyl)pyrazolo[1,5-*a*]pyridin-5-yl]benzamide
• 3-[2-(4-Chlorophenyl)pyrazolo[1,5-*a*]pyridin-5-yl]-*N*,*N*-dimethylbenzamide
• 3-[2-(4-Chlorophenyl)pyrazolo[1,5-*a*]pyridin-5-yl]-*N*-methylbenzamide
• Methyl 3-[2-(4-fluorophenyl)pyrazolo[1,5-*a*]pyridin-5-yl]benzoate
• 3-[2-(4-Fluorophenyl)pyrazolo[1,5-*a*]pyridin-5-yl]-*N*-methylbenzamide
• 3-[2-(4-Fluorophenyl)pyrazolo[1,5-*a*]pyridin-5-yl]-*N*-isopropylbenzamide
• 3-[2-(4-Fluorophenyl)pyrzolo[1,5-*a*]pyridin-5-yl]-*N*,*N*-dimethylbenzamide
• {3-[2-(4-Fluorophenyl)pyrazolo[1,5-*a*]pyridin-5-yl]phenyl}morpholin-4-yl-methanone
• 3-[2-(4-Fluorophenyl)pyrazolo[1,5-a]pyridin-5-yl]-2,*N*-dimethylbenzamide
• 2-Chloro-5-[2-(4-fluorophenyl)pyrazolo[1,5*-a*]pyridin-5-yl]-*N*-methylbenzamide
• 4-Chloro-3-[2-(4-fluorophenyl)pyrazolo[1,5-*a*]pyridin-5-yl]-*N*-methylbenzamide
• 3-[2-(4-Fluorophenyl}pyrazolo[1,5-*a*]pyridin-5-yl]-4,*N*-dimethylbenzamide
• 2-Fluoro-4-[2-(4-fluorophenyl)pyrazolo[1,5-*a*]pyridin-5-yl]-*N*-methylbenzamide
• *N*-Cyclopropyl-3-[2-(4-fluorophenyl)pyrazolo[1,5-*a*]pyridin-5-yl]benzamide
• {3-[2-(4-Fluorophenyl)pyrazolo[1,5-*a*]pyridin-5-yl]phenyllpyrrolidin-1-ylmethanone
• 3-[2-(2,6-Difluorophenyl)pyrazolo[1,5-*a*]pyridin-5-yl-methylbenzamide
• 3-[2-(2-Fluorophenyl)pyrazolo[1,5-a]pyridin-5-yl]-*N*-methylbenzamide
• *N*-Methyl-3-[2-(4-trifluoromethylphenyl)pyrazolo[1,5-*a*]pyridin-5-yl]benzamide
• 4-[2-(4-Fluorophenyl)pyrazolo[1,5-*a*]pyridin-5-yl]-*N*-methylbenzamide
• 2-[2-(4-Fluorophenyl)pyrazolo[1,5-*a*]pyridin-5-yl]-*N*-methylbenzamide
• {3-[2-(4-Fluorophenyl)pyrazolo[1,5-*a*]pyridin-5-yl]phenyl}-(3-hydroxypyrrolidin-1-yl)methanone
• 3-[2-(2,4-Difluorophenyl)pyrazolo[1,5-*a*]pyridin-5-yl]-*N*-methylbenzamide
• *N*-Methyl-3-[2-(4-trifluoromethoxyphenyl)pyrazolo[1,5-*a*]pyridin-5-yl]benzamide
• 3-[2-(3,4-Difluorophenyl)pyrazolo[1,5-*a*]pyrazolo-5-yl]-*N*-methylbenzamide
• 3-[2-(3,5-Difluorophenyl)pyrazolo[1,5-*a*]pyridin-5-yl]-*N*-methylbenzamide
• 3-[2-(3-Fluorophenyl)pyrazolo[1,5-*a*]pyridin-5-yl]-*N*-methylbenzamide
• *N*-Methyl-3-(2-p-tolylpyrazolo[1,5-*a*]pyridin-5-yl)benzamide
• 3-[2-(4-Methoxyphenyl)pyrazolo[1,5-*a*]pyridin-5-yl]-*N*-methylbenzamide
• 3-[2-(3,4-Dimethylphenyl)pyrazolo[1,5-a]pyridin-5-yl]-*N*-methylbenzamide
• 3-[2-(4-Cyanophenyl)pyrazolo[1,5-*a*]pyridin-5-yl]-*N*-methylbenzamide
• 3-[2-(2,3-Difluorophenyl)pyrazolo[1,5-*a*]pyridin-5-yl]-*N*-methylbenzamide
• *N*-Methyl-3-(2-*o*-tolylpyrazolo[1,5-*a*]pyridin-5-yl)benzamide
• 3-[3-Chloro-2-(4-fluorophenyl)pyrazolo[1,5-*a*]pyridin-5-yl]-*N*-methylbenzamide

7. Medicament, **characterized in that** it comprises a compound of formula (I) according to any one of Claims 1 to 6, or an addition salt of this compound with a pharmaceutically acceptable acid.

8. Pharmaceutical composition, **characterized in that** it comprises a compound of formula (I) according to any one of Claims 1 to 6, or a pharmaceutically acceptable salt of this compound, and also at least one pharmaceutically acceptable excipient.

9. Use of a compound of formula (I) according to any one of Claims 1 to 6, for the preparation of a medicament for treating and preventing neurodegenerative diseases.

10. Use of a compound of formula (I) according to any one of Claims 1 to 6, for the preparation of a medicament for treating and preventing cerebral trauma and epilepsy.

11. Use of a compound of formula (I) according to any one of Claims 1 to 6, for the preparation of a medicament for treating and preventing psychiatric diseases.

12. Use of a compound of formula (I) according to any one of Claims 1 to 6, for the preparation of a medicament for treating and preventing inflammatory diseases.

13. Use of a compound of formula (I) according to any one of Claims 1 to 6, for the preparation of a medicament for treating and preventing osteoporosis and cancers.

14. Use of a compound of formula (I) according to any one of Claims 1 to 6, for the preparation of a medicament for treating and preventing Parkinson's disease, Alzheimer's disease, tauopathies and multiple sclerosis.

15. Use of a compound of formula (I) according to any one of Claims 1 to 6, for the preparation of a medicament for treating and preventing schizophrenia, depression, substance dependency and attention-deficit hyperactivity disorder.

16. Intermediate compound of formula (VI-1)

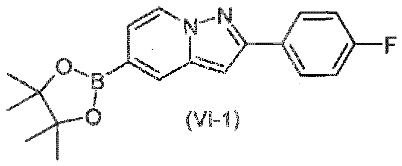

17. Use of the compound according to Claim 16 for the synthesis of products of general formula (I) according to Claim 1.
